# EUROPEAN PATENT SPECIFICATION

(11) **EP 3 757 603 B1**
(45) Date of publication and mention of the grant of the patent: **19.02.2025**
(21) Application number: 19757730.7
(22) Date of filing: 19.02.2019
(51) Int. Cl.: G01S 7/481, G01S 17/08, G01S 17/89, G01S 17/931, A61B 1/00, G02B 5/20, G02B 5/22, G02B 5/28, G01S 7/4865, A61B 1/04, A61B 1/045, A61B 1/313, B29D 11/00, B29C 51/10, A61B 1/06, A61B 1/07

(54) **RANGING SYSTEM, LIGHT RECEIVING MODULE, AND METHOD FOR MANUFACTURING BAND-PASS FILTER**
ENTFERNUNGSMESSSYSTEM, LICHTEMPFANGSMODUL UND VERFAHREN ZUR HERSTELLUNG EINES BANDPASSFILTERS
SYSTÈME DE TÉLÉMÉTRIE, MODULE DE RÉCEPTION DE LUMIÈRE ET PROCÉDÉ DE FABRICATION D'UN FILTRE PASSE-BANDE

(30) Priority: 21.02.2018 JP 2018028508; 22.01.2019 WO PCT/JP2019/001822
(43) Date of publication of application: 30.12.2020
(73) Proprietor: Sony Semiconductor Solutions Corporation, Atsugi-shi, Kanagawa 243-0014 (JP)
(72) Inventor: YOKOGAWA Sozo, Atsugi-shi, Kanagawa 243-0014 (JP); KIKUCHI Yuki, Atsugi-shi, Kanagawa 243-0014 (JP); SUWA Taisuke, Atsugi-shi, Kanagawa 243-0014 (JP); CHIYODA Makoto, Atsugi-shi, Kanagawa 243-0014 (JP)
(74) Representative: Müller Hoffmann & Partner
(86) International application number: PCT/JP2019/006064
(87) International publication number: WO 2019/163761

(56) References cited:
- JP-A- 2009 145 779
- JP-A- 2016 218 893
- US-A1- 2007 057 162
- US-A1- 2009 257 021
- US-A1- 2012 133 799
- US-A1- 2012 133 799
- US-A1- 2014 185 134
- US-A1- 2018 055 325

## Description

### TECHNICAL FIELD

The present disclosure relates to a distance measuring system, a light receiving module, and a method of manufacturing a bandpass filter.

### BACKGROUND ART

In recent years, a distance measuring system has been proposed in which information regarding a distance to a target object is obtained by emitting light to the target object and receiving the reflected light (for example, see Patent Document 1). The configuration of emitting infrared light and receiving the reflected light to obtain distance information has advantages, for example, a light source is not very noticeable, and an operation can be performed in parallel with capturing a normal visible light image.

In terms of reducing disturbance that affects measurement, it is preferable to limit a wavelength range of infrared light, which is the electromagnetic wavelength to be imaged, as narrowly as possible. For this reason, a bandpass filter that is transparent to only a specific wavelength band is often arranged in front of an imaging element.

US 2012/133799 A1 describes a radiation sensor including a radiation source, first and second pixels with a radiation absorption filter positioned over the first pixel, an interference filter positioned over both the first and second pixels, and a controller. The interference filter is selectively transparent to infrared light in a predetermined wavelength range and has a concave-shaped light incident surface. The radiation sensor may be used to detect the proximity of an object. This is done by activating the radiation source to emit radiation, detecting a portion of the radiation reflected by the object at the first pixel of the sensor, and processing the reflected radiation with the controller to calculate the proximity of the object, for example by using a direct time of flight measurement.

### CITATION LIST

### PATENT DOCUMENT

Patent Document 1: Japanese Patent Application Laid-Open No. 2017-150893

### SUMMARY OF THE INVENTION

### PROBLEMS TO BE SOLVED BY THE INVENTION

In order to cope with a reduction in height of housings of electronic equipment, light receiving modules and the like used in portable electronic equipment are compelled to have a configuration of an optical system with so-called pupil correction, in which a chief ray angle differs greatly between the center and the periphery of the imaging element. Band characteristics of a bandpass filter shift in a wavelength direction depending on an angle of incident light. Therefore, in order to receive target light at the center and the periphery of a light receiving unit including an imaging element and the like without any trouble, it is necessary to set a bandwidth of the bandpass filter to be wider than a normal bandwidth. This causes an influence of disturbance light to increase.

It is therefore an object of the present disclosure to provide a distance measuring system, a light receiving module, and a method of manufacturing a bandpass filter that enables setting a narrow bandwidth for the bandpass filter and reducing the influence of disturbance light.

### SOLUTIONS TO PROBLEMS

The invention is defined by the appended claims.

To achieve the above-described object, a distance measuring system according to the invention defined in claim 1 is provided.

### BRIEF DESCRIPTION OF DRAWINGS

Fig. 1 is a schematic diagram illustrating a basic configuration of a distance measuring system according to a first embodiment of the present disclosure.
Fig. 2 is a schematic diagram illustrating a configuration of an optical member in a distance measuring system of a reference example not being part of the invention.
Fig. 3A is a schematic graph illustrating a relationship between an image height and an angle with respect to a chief ray angle (CRA) in the optical member of the reference example. Fig. 3B is a schematic graph illustrating characteristics of a bandpass filter in the optical member of the reference example.
Fig. 4A is a schematic diagram illustrating a configuration of an optical member in the distance measuring system according to the first embodiment. Fig. 4B is a schematic graph illustrating characteristics of a bandpass filter in the optical member according to the first embodiment.
Fig. 5 is a schematic graph illustrating a relationship between a wavelength shift and an angle with respect to a CRA in the bandpass filter.
Figs. 6A and 6B are schematic diagrams illustrating a configuration of the bandpass filter. Fig. 6C is a schematic graph illustrating the characteristics of the bandpass filter.
Fig. 7A is a schematic graph illustrating characteristics of a first filter. Fig. 7B is a schematic graph illustrating characteristics of a second filter.
Fig. 8 is a diagram illustrating a configuration example of the first filter, and Fig. 8A is a table illustrating a stacking relationship. Fig. 8B illustrates transmission characteristics of the filter.
Fig. 9 is a diagram illustrating a configuration example of the second filter, and Fig. 9A is a table illustrating a stacking relationship. Fig. 9B illustrates transmission characteristics of the filter.
Figs. 10A, 10B, 10C, and 10D are schematic diagrams illustrating a first method of manufacturing a bandpass filter.
Figs. 11A, 11B, 11C, and 11D are schematic diagrams illustrating a second method of manufacturing a bandpass filter.
Figs. 12A, 12B, and 12C are schematic diagrams illustrating another configuration example of a bandpass filter.
Figs. 13A, 13B, 13C, and 13D are schematic diagrams illustrating a third method of manufacturing a bandpass filter.
Figs. 14A, 14B, 14C, and 14D are schematic diagrams illustrating a fourth method of manufacturing a bandpass filter.
Fig. 15 is a schematic diagram illustrating a configuration of a sheet material used in a fifth method of manufacturing a bandpass filter.
Figs. 16A, 16B, and 16C are schematic diagrams illustrating vacuum forming in the fifth method of manufacturing a bandpass filter.
Fig. 17 is a schematic diagram illustrating press working in the fifth method of manufacturing a bandpass filter.
Figs. 18A and 18B are schematic diagrams illustrating a method of manufacturing a light receiving module.
Figs. 19A and 19B are schematic diagrams illustrating a structure of a light receiving module.
Fig. 20 is a schematic diagram illustrating a structure of a light receiving module including a lens.
Figs. 21A, 21B, and 21C are schematic diagrams illustrating a configuration of a semiconductor device used in the distance measuring system.
Fig. 22 is a schematic diagram illustrating a first modified example of the distance measuring system.
Fig. 23 is a schematic diagram illustrating a second modified example of the distance measuring system.
Fig. 24 is a schematic diagram illustrating a third modified example of the distance measuring system.
Fig. 25 is a schematic diagram illustrating a fourth modified example of the distance measuring system.
Figs. 26A and 26B are schematic diagrams illustrating an example of arrangement of a light receiving unit and a light source unit in portable electronic equipment.
Fig. 27 is a block diagram illustrating an example of a schematic configuration of a vehicle control system.
Fig. 28 is an explanatory diagram illustrating an example of installation positions of an outside-of-vehicle information detector and an imaging unit.
Fig. 29 is a diagram illustrating an example of a schematic configuration of an endoscopic surgery system.
Fig. 30 is a block diagram illustrating an example of a functional configuration of a camera head and a CCU illustrated in Fig. 29.

### MODE FOR CARRYING OUT THE INVENTION

The present disclosure will be described below with reference to the drawings on the basis of an embodiment. The present disclosure is not limited to the embodiment, and the various numerical values, materials, and the like in the embodiment are examples. In the following description, the same elements or elements having the same functions will be denoted by the same reference numerals, without redundant description. Note that the description will be made in the order below.
1. Overall description of distance measuring system and light receiving module according to present disclosure
2. First embodiment
3. First modified example
4. Second modified example
5. Third modified example
6. Fourth modified example
7. First application example
8. Second application example
9. Configuration of present disclosure

### [Overall description of distance measuring system and light receiving module according to present disclosure]

As described above, a distance measuring system according to the present disclosure includes:
a light source unit that emits infrared light toward a target object;
a light receiving unit that receives the infrared light from the target object; and
an arithmetic processing unit that obtains information regarding a distance to the target object on the basis of data from the light receiving unit,
in which an optical member including a bandpass filter that is selectively transparent to infrared light in a predetermined wavelength range is arranged on a light receiving surface side of the light receiving unit, and
the bandpass filter has a concave-shaped light incident surface.

The distance measuring system according to the present disclosure may have a configuration in which
the optical member includes a lens arranged on a light incident surface side of the bandpass filter, and
an incident angle of light at a maximum image height with respect to the light incident surface of the bandpass filter is 10 degrees or less.

The distance measuring system of the present disclosure including the preferable configuration described above may have a configuration in which
a transmission band of the bandpass filter has a half-width of 50 nm or less.

The distance measuring system of the present disclosure including the various preferable configurations described above may have a configuration in which
the bandpass filter includes
a first filter that is transparent to light in a predetermined wavelength range of infrared light, and
a second filter that is non-transparent to visible light and transparent to infrared light.

In this case,
the first filter and the second filter may be stacked and formed on one side of a base material
in the configuration. Alternatively,
the first filter may be formed on one surface of a base material, and
the second filter may be formed on another surface of the base material
in the configuration.

The distance measuring system of the present disclosure including the various preferable configurations described above may have a configuration in which
the first filter is arranged on the light incident surface side, and
the second filter is arranged on a light receiving unit side.

In this case, the second filter may have a concave shape that imitates the light incident surface in the configuration. Alternatively, the second filter may have a planar shape in the configuration.

Alternatively,
the second filter may be arranged on the light incident surface side, and
the first filter may be arranged on the light receiving unit side
in the configuration.

In this case, the first filter may have a concave shape that imitates the light incident surface in the configuration.

The distance measuring system of the present disclosure including the various preferable configurations described above may have a configuration in which
the light source unit includes an infrared laser element or an infrared light emitting diode element.

The distance measuring system of the present disclosure including the various preferable configurations described above may have a configuration in which
the light source unit emits infrared light having a center wavelength of approximately 850 nm, approximately 905 nm, or approximately 940 nm.

The distance measuring system of the present disclosure including the various preferable configurations described above may have a configuration in which
the arithmetic processing unit obtains distance information on the basis of a time of flight of light reflected from the target object.

Alternatively,
infrared light may be emitted in a predetermined pattern to the target object, and
the arithmetic processing unit may obtain distance information on the basis of a pattern of light reflected from the target object
in the configuration.

As described above, a light receiving module according to the present disclosure includes:
a light receiving unit that receives infrared light; and
an optical member that is arranged on a light receiving surface side of the light receiving unit and includes a bandpass filter that is selectively transparent to infrared light in a predetermined wavelength range,
in which the bandpass filter has a concave-shaped light incident surface.

The light receiving module according to the present disclosure may have a configuration in which
the optical member includes a lens arranged on a light incident surface side of the bandpass filter. In this case, an incident angle of light at a maximum image height with respect to the light incident surface of the bandpass filter may be 10 degrees or less in the configuration.

As described above, a method of manufacturing a bandpass filter according to the present disclosure includes:
forming a bandpass filter layer on a film sheet that is transparent to at least an infrared light component and subject to plastic deformation;
placing the film sheet on which the bandpass filter layer has been formed, on a mold in which a concave portion is formed on one surface and an opening that passes through from the concave portion to another surface is formed; and
sucking air in the concave portion from the other surface through the opening.

The method of manufacturing a bandpass filter according to the present disclosure may have a configuration in which
the film sheet, on which the bandpass filter layer has been formed, is singulated into a predetermined shape including a concave surface formed by sucking the air in the concave portion.

In the distance measuring system and the light receiving module of the present disclosure including the various preferable configurations described above, for example, a photoelectric conversion element or an imaging element such as a CMOS sensor or a CCD sensor in which pixels including various pixel transistors are arranged in a two-dimensional matrix in a row direction and a column direction may be used as the light receiving unit.

In the distance measuring system of the present disclosure including the various preferable configurations described above may have a configuration in which the arithmetic processing unit that obtains information regarding the distance to the target object on the basis of data from the light receiving unit operates on the basis of physical connection by hardware, or operates on the basis of a program. The same applies to a controller that controls the entire distance measuring system, and the like.

### [First embodiment]

A first embodiment relates to a distance measuring system and a light receiving module according to the present disclosure.

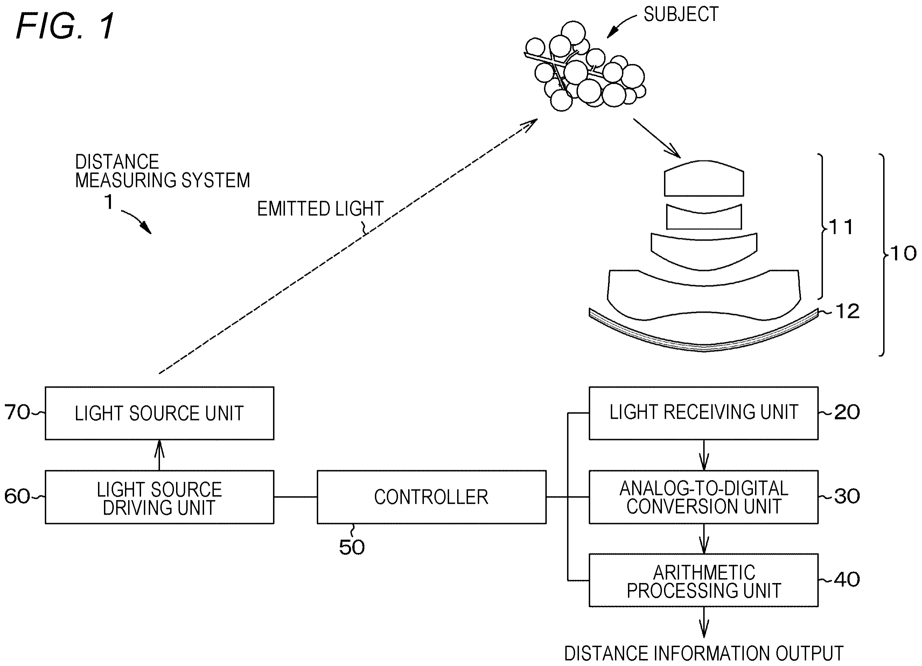

Fig. 1 is a schematic diagram illustrating a basic configuration of the distance measuring system according to the first embodiment of the present disclosure.

A distance measuring system 1 includes:
a light source unit 70 that emits infrared light toward a target object;
a light receiving unit 20 that receives the infrared light from the target object; and
an arithmetic processing unit 40 that obtains information regarding a distance to the target object on the basis of data from the light receiving unit 20.

On a light receiving surface side of the light receiving unit 20, an optical member 10 including a bandpass filter 12 that is selectively transparent to infrared light in a predetermined wavelength range is arranged. The bandpass filter 12 has a concave-shaped light incident surface. The optical member 10 includes lenses (lens group) 11 arranged on a light incident surface side of the bandpass filter 12.

The light receiving unit 20 is constituted by a CMOS sensor or the like, and a signal of the light receiving unit 20 is digitized by an analog-to-digital conversion unit 30 and sent to the arithmetic processing unit 40. These operations are controlled by a controller 50.

The light source unit 70 emits, for example, infrared light having a wavelength in a range of about 700 to 1100 nm. The light source unit 70 includes a light emitting element such as an infrared laser element or an infrared light emitting diode element. The deviation from the center wavelength is about 1 nm for the former and about 10 nm for the latter. The light source unit 70 is driven by a light source driving unit 60 controlled by the controller 50.

The wavelength of the infrared light emitted by the light source unit 70 can be appropriately selected depending on the intended use and configuration of the distance measuring system. For example, a value such as approximately 850 nm, approximately 905 nm, or approximately 940 nm can be selected as the center wavelength.

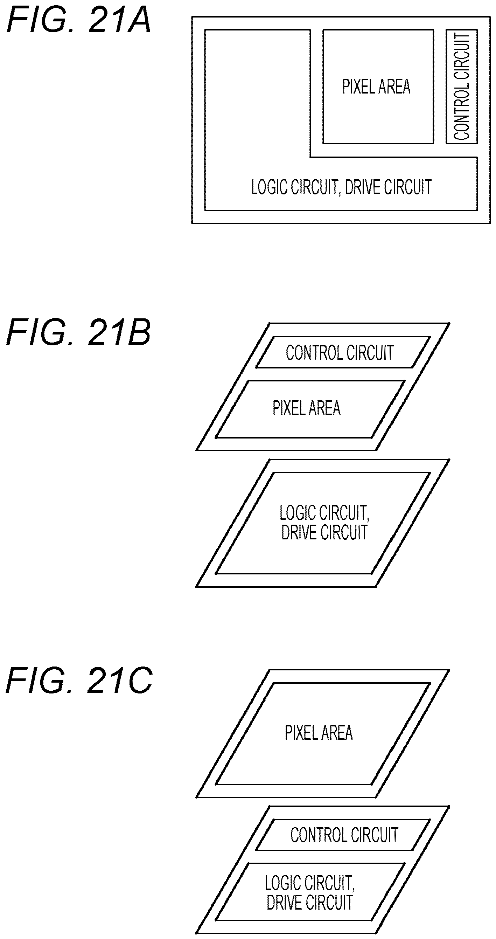

The light receiving unit 20, the analog-to-digital conversion unit 30, the arithmetic processing unit 40, the controller 50, and the light source driving unit 60 are formed on a semiconductor substrate including, for example, silicon. They may be configured as a single chip, or may be configured as a plurality of chips in accordance with their functions. This will be described with reference to Fig. 21A described later.

A receiving system 1 may be configured as a unit so as to be suitable for, for example, being built in equipment, or may be configured separately.

The basic configuration of the distance measuring system 1 has been described above. Next, in order to facilitate understanding of the present disclosure, a reference example of a configuration in which a bandpass filter has a planar light incident surface, and a problem thereof will be described.

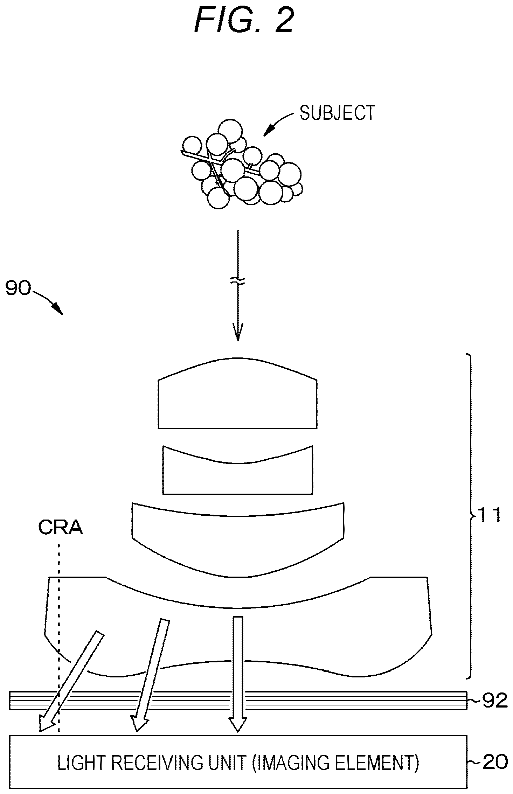

Fig. 2 is a schematic diagram illustrating a configuration of an optical member in a distance measuring system of the reference example.

An optical member 90 of the reference example differs from the optical member 10 illustrated in Fig. 1 in that the optical member 90 has a planar bandpass filter 92.

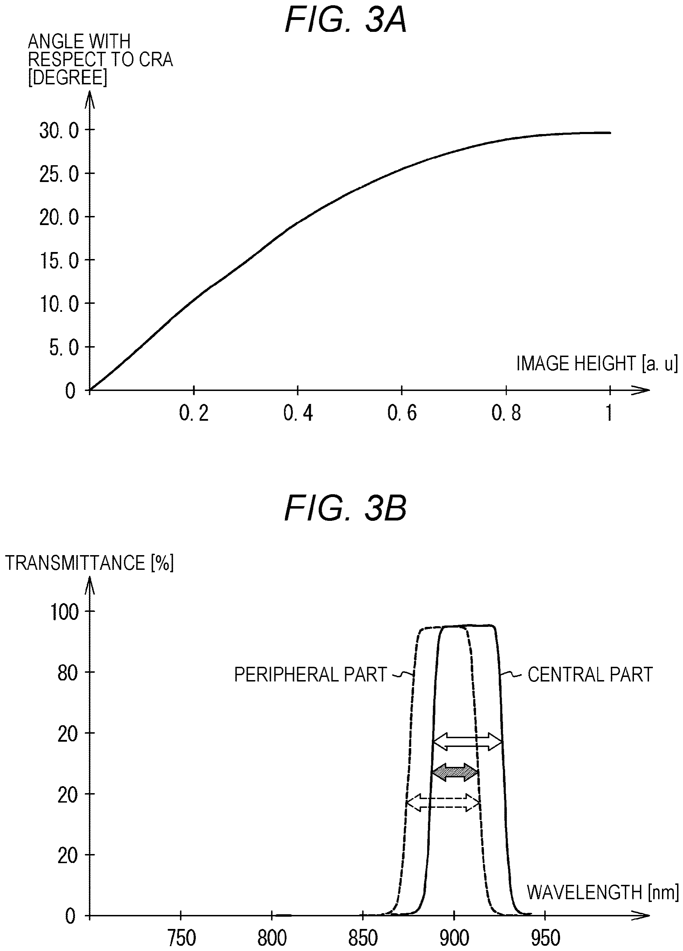

Fig. 3A is a schematic graph illustrating a relationship between an image height and an angle with respect to a chief ray angle (CRA) in the optical member of the reference example. Fig. 3B is a schematic graph illustrating characteristics of a bandpass filter in the optical member of the reference example.

For example, in a case where a lens is configured so as to cope with a reduction in height, the lens is compelled to have a configuration in which the chief ray angle differs greatly between a central part and a peripheral part of the light receiving unit 20. Fig. 3A illustrates the relationship between the image height and the angle with respect to the CRA in such a case. The graph is normalized on the basis of a case where the image height at the light receiving unit 20 is maximum (which normally corresponds to four corners of a screen). As illustrated in the graph, as compared to a case where the image height is 0, the angle with respect to the CRA changes by about 30 degrees in a case where the image height is the maximum.

As a result, in a case where light is incident on the central part of the light receiving unit 20 and in a case where light is incident on the peripheral part, the incident angle of light with respect to the bandpass filter 92 also changes by about 30 degrees. In a case where light is obliquely incident on the bandpass filter 92, the optical path length of the light passing through the filter increases, so that the characteristics shift toward a short wavelength side.

Thus, for example, in a case where the reception target is infrared light having a center wavelength of 905 nm, it is necessary to set the band center of the bandpass filter 92 in a case where the angle with respect to the CRA is 0 to a wavelength longer than 905 nm. Furthermore, the bandwidth also needs to be set so as to enable transmission of 905 nm even in a case where the angle with respect to the CRA is 0 degrees to 30 degrees. As a result, the bandwidth of the bandpass filter 92 needs to be set wider than a normal bandwidth. This causes an increase in the influence of disturbance such as inclusion of ambient light.

The reference example of the configuration in which the bandpass filter has a planar light incident surface and the problem thereof have been described above.

Subsequently, the first embodiment will be described.

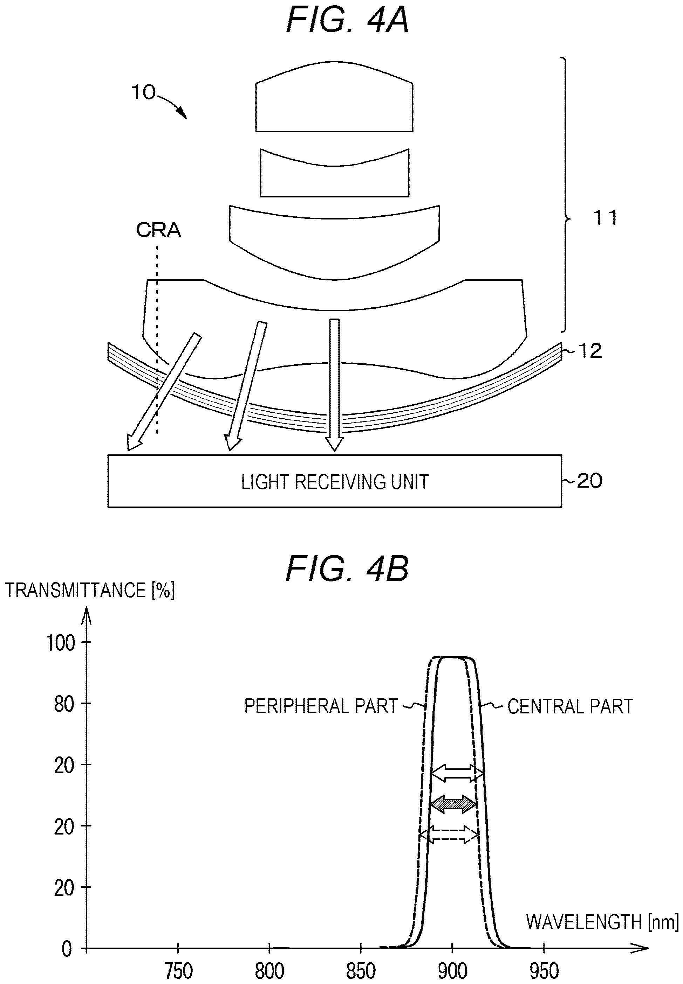

Fig. 4A is a schematic diagram illustrating a configuration of an optical member in the distance measuring system according to the first embodiment. Fig. 4B is a schematic graph illustrating characteristics of a bandpass filter in the optical member according to the first embodiment.

As illustrated in Fig. 4A, the bandpass filter 12 in the first embodiment has a concave-shaped light incident surface. With this arrangement, a change in the incident angle of light with respect to the bandpass filter 12 is reduced.

Thus, for example, in a case where the reception target is infrared light having a center wavelength of 905 nm, the band center of the bandpass filter 12 in a case where the angle with respect to the CRA is 0 can be set to be close to 905 nm. Furthermore, even in a case where light is incident on the peripheral part of the light receiving unit 20, the amount of shift of the characteristic of the bandpass filter 12 toward the short wavelength side is reduced. As a result, the bandwidth of the bandpass filter 92 can be set to be narrower, and the influence of disturbance can be suppressed. With this arrangement, measurement accuracy can be improved.

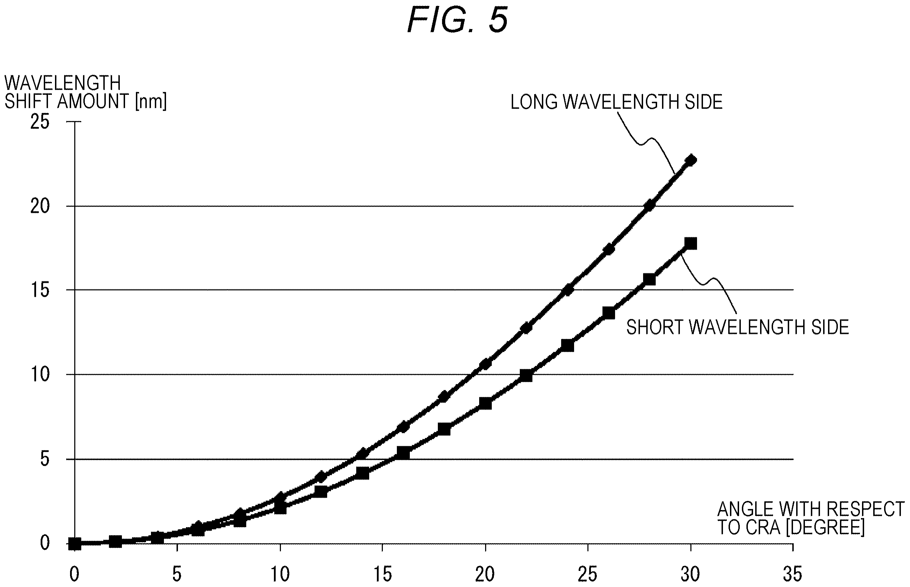

Fig. 5 is a schematic graph illustrating a relationship between a wavelength shift and the angle with respect to the CRA in the bandpass filter. More specifically, the amount of shift of the value on the short wavelength side and that of the value on the long wavelength side of a transmission band of the bandpass filter 12 are illustrated.

According to Fig. 5, in a case where the angle with respect to the CRA is about 30 degrees, the transmission band of the bandpass filter 12 shifts by about 20 nm. On the other hand, in a case where the angle with respect to the CRA is about 10 degrees, the shift amount of the transmission band can be suppressed to about one-tenth. Thus, it is preferable to set the shape of the bandpass filter 12 so that the incidence angle of light at a maximum image height with respect to the light incident surface of the bandpass filter 12 is 10 degrees or less. Furthermore, the transmission band of the bandpass filter 12 preferably has a half-width of 50 nm or less.

The bandpass filter 12 may have a configuration including a first filter that is transparent to light in a predetermined wavelength range of infrared light, and a second filter that is non-transparent to visible light and transparent to infrared light. A configuration example and a manufacturing method of the bandpass filter 12 will be described below with reference to the drawings.

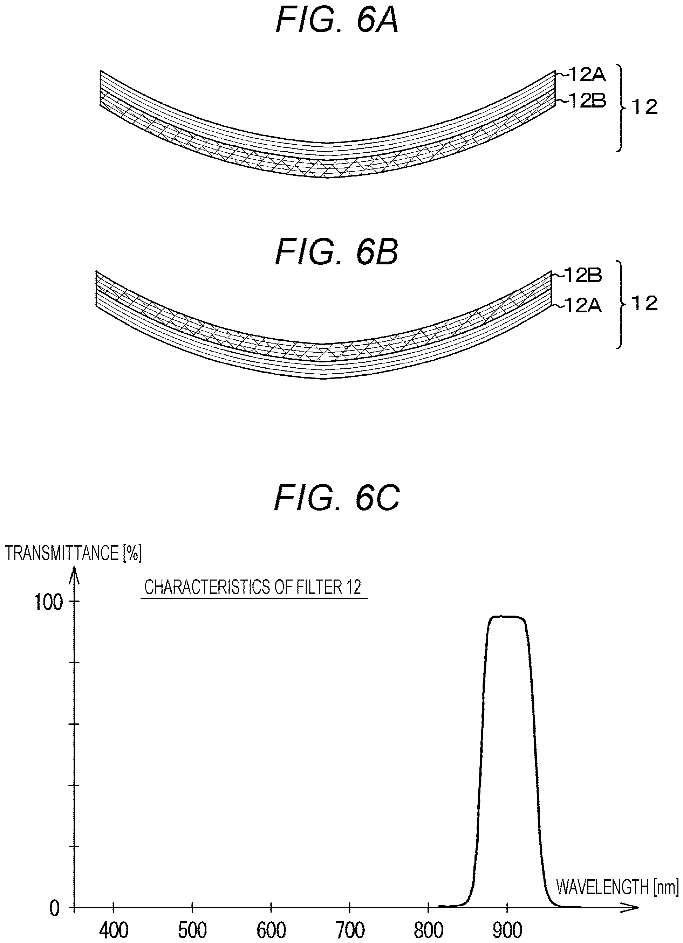

Figs. 6A and 6B are schematic diagrams illustrating a configuration of the bandpass filter. Fig. 6C is a schematic graph illustrating the characteristics of the bandpass filter.

Fig. 6A illustrates a configuration example in which a first filter 12A is arranged on the light incident surface side, and a second filter 12B is arranged on a light receiving unit 20 side. Fig. 6B illustrates a configuration example in which the second filter 12B is arranged on the light incident surface side, and the first filter 12A is arranged on the light receiving unit 20 side. Both show transmission characteristics as illustrated in Fig. 6C.

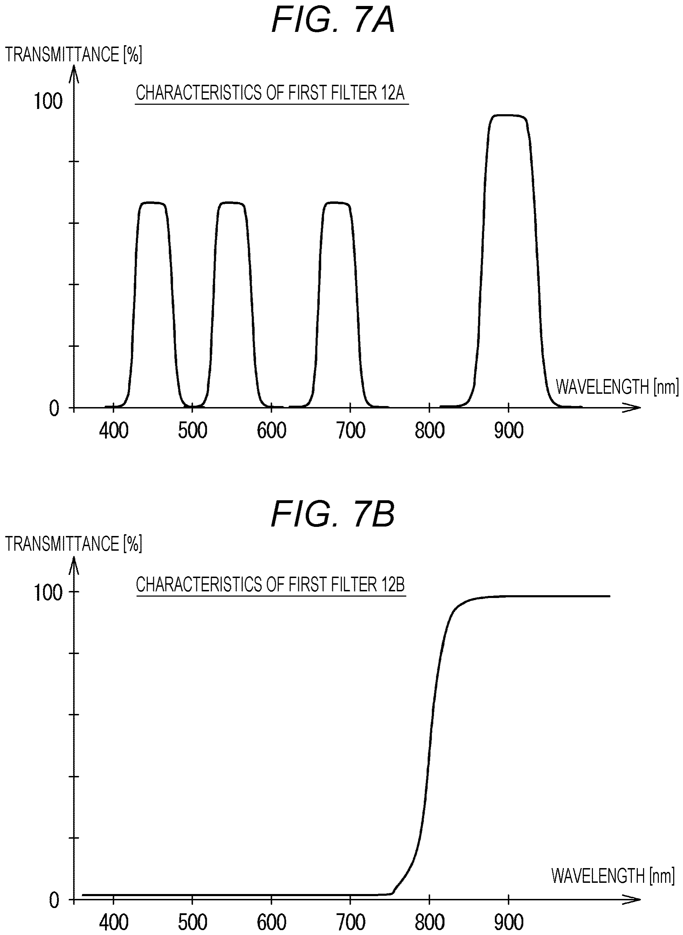

Fig. 7A is a schematic graph illustrating characteristics of the first filter. Fig. 7B is a schematic graph illustrating characteristics of the second filter.

An optical filter can be constituted by, for example, a multilayer film in which a high refractive index material and a low refractive index material are appropriately stacked. However, in a case where the optical filter is designed so that the wavelength band including target light may have transmission characteristics, even light having, for example, a frequency that has a multiplication relationship exhibits some transmission characteristics. Thus, the characteristics of the first filter 12A are schematically represented as illustrated in Fig. 7A. For this reason, as illustrated in Fig. 7B, the second filter 12B that is non-transparent to visible light and transparent to infrared light is also included. As a result, characteristics of the entire filter are as illustrated in Fig. 6C.

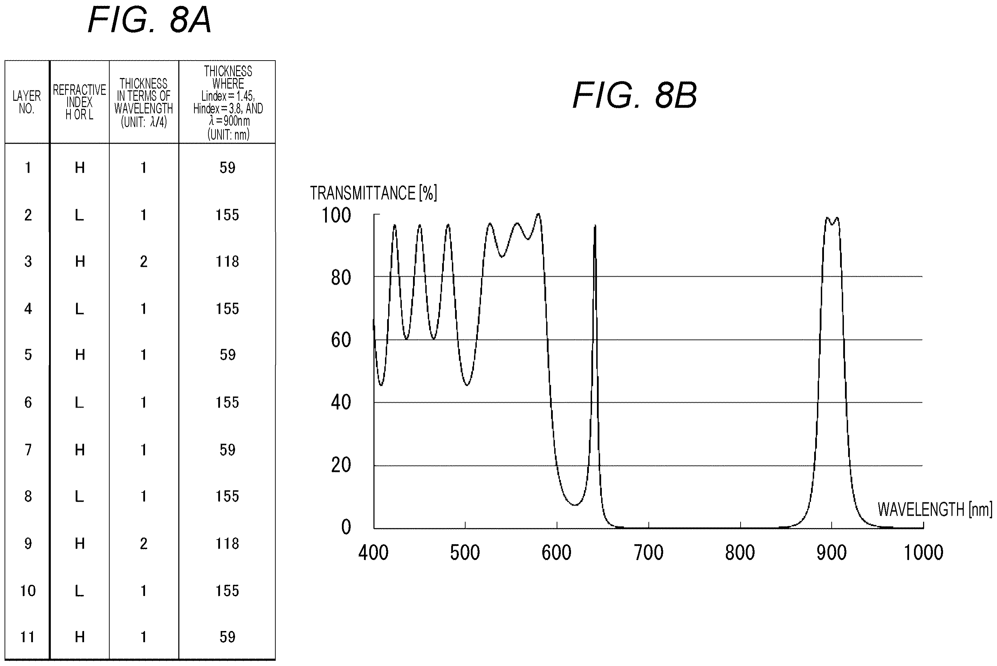

Fig. 8 is a diagram illustrating a configuration example of the first filter, and Fig. 8A is a table illustrating a stacking relationship. Fig. 8B illustrates transmission characteristics of the filter.

In this example, the first filter 12A is constituted by an eleven-layer multilayer film. Silicon oxide is used as the high refractive index material, and silicon is used as the low refractive index material.

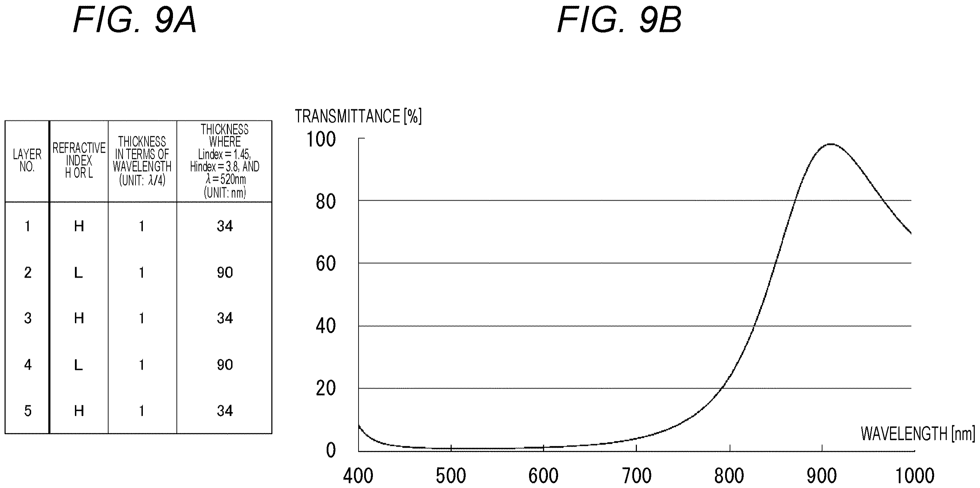

Fig. 9 is a diagram illustrating a configuration example of the second filter, and Fig. 9A is a table illustrating a stacking relationship. Fig. 9B illustrates transmission characteristics of the filter.

In this example, the second filter 12B is constituted by a five-layer multilayer film. Silicon oxide is used as the high refractive index material, and silicon is used as the low refractive index material.

A known method such as CVD, PDV, or ALD can be used as a method of forming a multilayer film, and it is preferable to select an ALD having advantages such as high-precision film formation and good coverage.

The first filter 12A and the second filter 12B may have a configuration in which they are stacked and formed on one side of a base material. The manufacturing method will be described below.

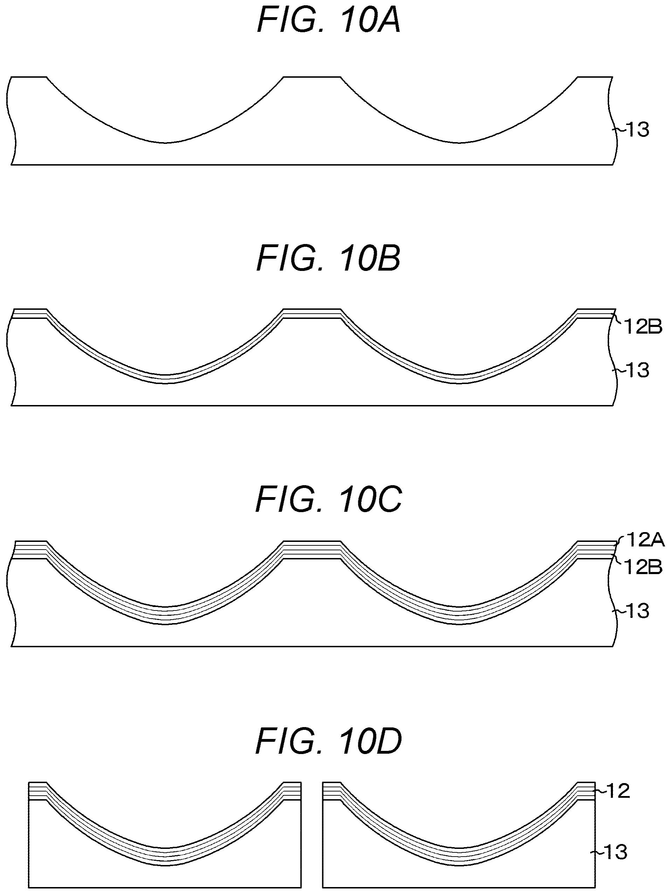

Figs. 10A, 10B, 10C, and 10D are schematic diagrams illustrating a first method of manufacturing a bandpass filter.

A base material 13 constituted by a material transparent to infrared light and having a concave formed on a surface is prepared (see Fig. 10A), and the second filter 12B constituted by a multilayer film is form thereon (see Fig. 10B). Next, the first filter 12A constituted by a multilayer film is formed thereon (see Fig. 10C). Thereafter, the bandpass filter 12 can be obtained by singulation into a predetermined shape including a concave (see Fig. 10D).

Note that, in the above-described example, the second filter 12B is formed, and then the first filter 12A is formed. However, a configuration in which the two are interchanged may be adopted.

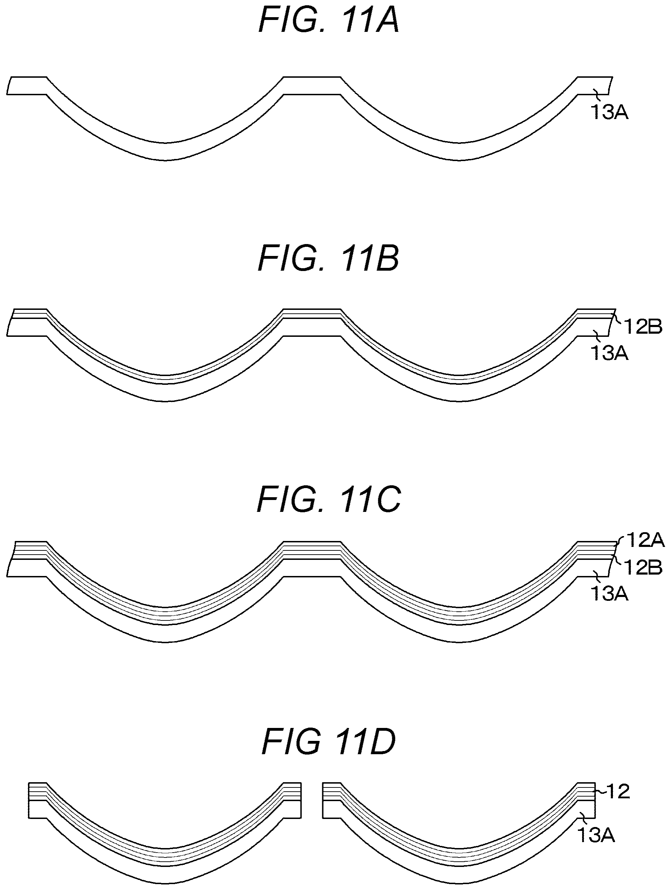

Figs. 11A, 11B, 11C, and 11D are schematic diagrams illustrating a second method of manufacturing a bandpass filter.

Except for a difference that a base material 13A having a concave formed on a front surface and having a convex on a corresponding back surface portion is used, this example is similar to the process flow described with reference to Fig. 10, and the description thereof will be omitted.

In the above-described configuration, the first filter 12A and the second filter 12B are stacked, but another configuration may also be used. For example, in such a configuration, the first filter 12A is formed on one surface of a base material, and the second filter 12B is formed on the other surface of the base material.

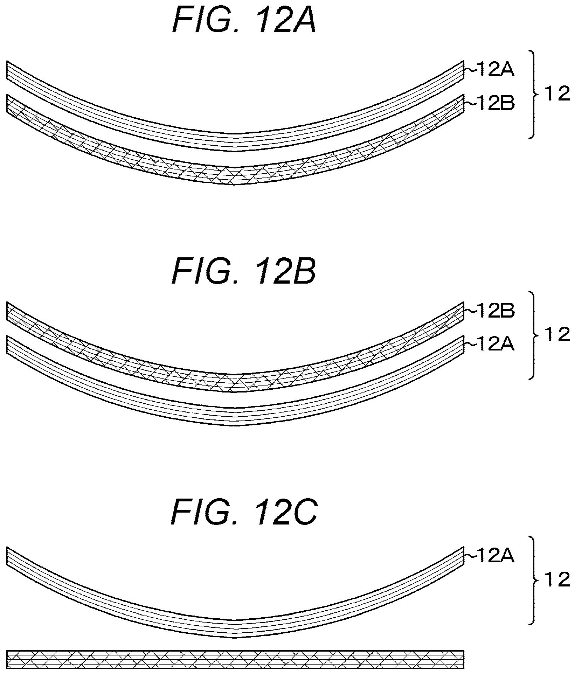

Figs. 12A, 12B, and 12C are schematic diagrams illustrating another configuration example of a bandpass filter.

In Figs. 12A and 12B, the first filter 12A and the second filter 12B are arranged at a fixed interval. In Fig. 12A, the first filter 12A is arranged on the light incident surface side, and the second filter 12B is arranged on the light receiving unit 20 side. On the other hand, in Fig. 12B, the second filter 12B is arranged on the light incident surface side, and the first filter 12A is arranged on the light receiving unit 20 side. Fig. 12C is a modification of Fig. 12A, and the second filter 12B is planar.

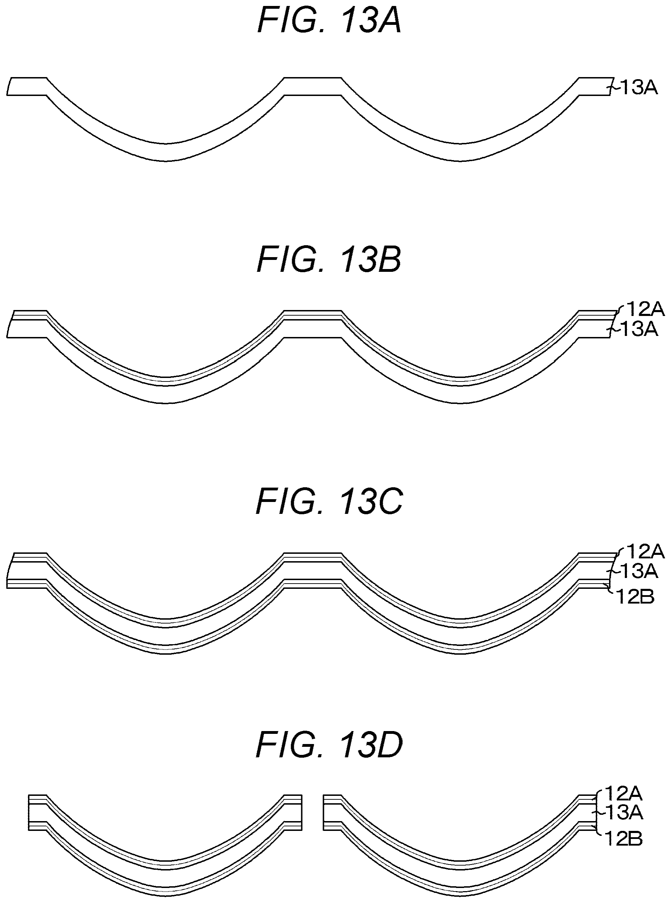

Figs. 13A, 13B, 13C, and 13D are schematic diagrams illustrating a third method of manufacturing a bandpass filter.

The base material 13A having a concave formed on the front surface and having a convex on the corresponding back surface portion is prepared (see Fig. 13A), and the first filter 12A constituted by a multilayer film is formed on the front surface (see Fig. 13B). Next, the second filter 12B constituted by a multilayer film is formed on the back surface of the base material 13A (see Fig. 13C). Thereafter, the bandpass filter 12 can be obtained by singulation into a predetermined shape including a concave surface (see Fig. 13D).

Note that, in the above-described example, the second filter 12B is formed, and then the first filter 12A is formed. However, a configuration in which the two are interchanged may be adopted.

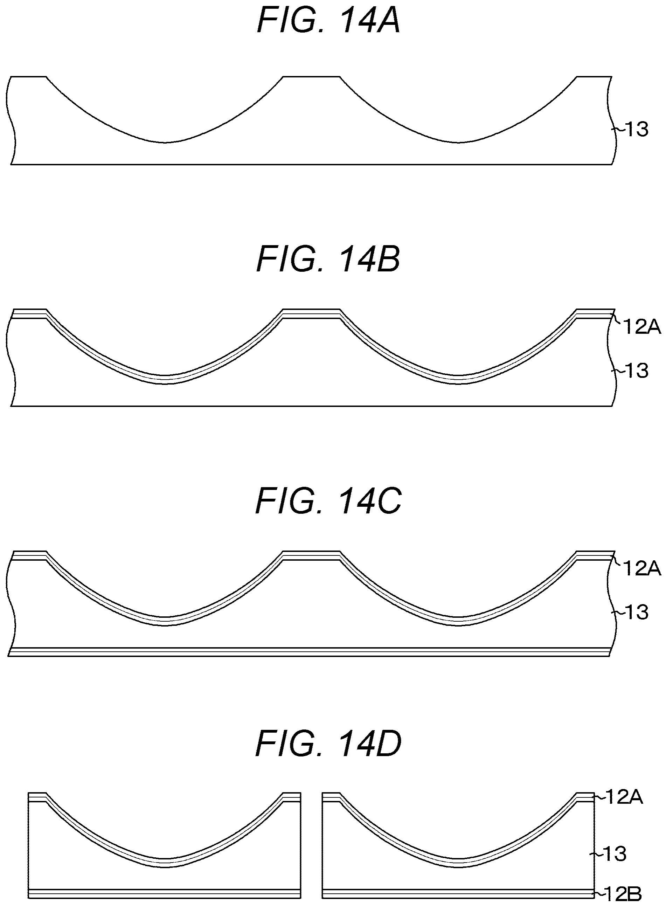

Figs. 14A, 14B, 14C, and 14D are schematic diagrams illustrating a fourth method of manufacturing a bandpass filter.

Except for a difference that the base material 13 having a concave formed on a front surface and having a flat back surface is used, this example is similar to the process flow described with reference to Fig. 14, and the description thereof will be omitted.

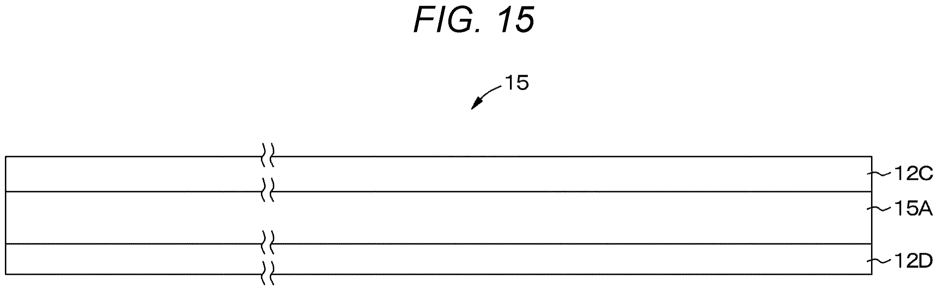

Figs. 15, 16A, 16B, 16C, and 17 are drawings illustrating a fifth method of manufacturing a bandpass filter.

Fig. 15 is a schematic diagram illustrating a configuration of a film sheet 15 used in the fifth method of manufacturing a bandpass filter. A film sheet 15A constituted by a material that is transparent to at least an infrared light component and plastically deformed when an external force is applied is prepared, and a reflective film 12C (bandpass filter layer, or BPF layer) is formed on one surface of the film sheet 15A by vapor deposition. Next, an antireflection film 12D (AR layer) is formed on the other surface of the film sheet 15A by vapor deposition. With this arrangement, the film sheet 15 on which the bandpass filter layer and the like are formed can be obtained.

Note that the antireflection film 12D may be vapor-deposited on the film sheet 15A first, and then the reflective film 12C may be vapor-deposited. Furthermore, the film sheet 15A has a bandpass filter function obtained by kneading an absorbing material. Specifically, an absorbing material is kneaded into or vapor-deposited on a material based on a resin-based sheet such as cycloolefin polymer, polyethylene terephthalate (PET), or polycarbonate to obtain the film sheet having bandpass characteristics. With this configuration, light in a wavelength band, which has not been able to be removed only by a reflective film vapor-deposited on one surface of a film sheet, can be removed by the film sheet having the bandpass characteristics. Note that the film sheet 15A is not limited to the configuration in the present disclosure, and a film sheet material having no band-pass characteristics may be applied.

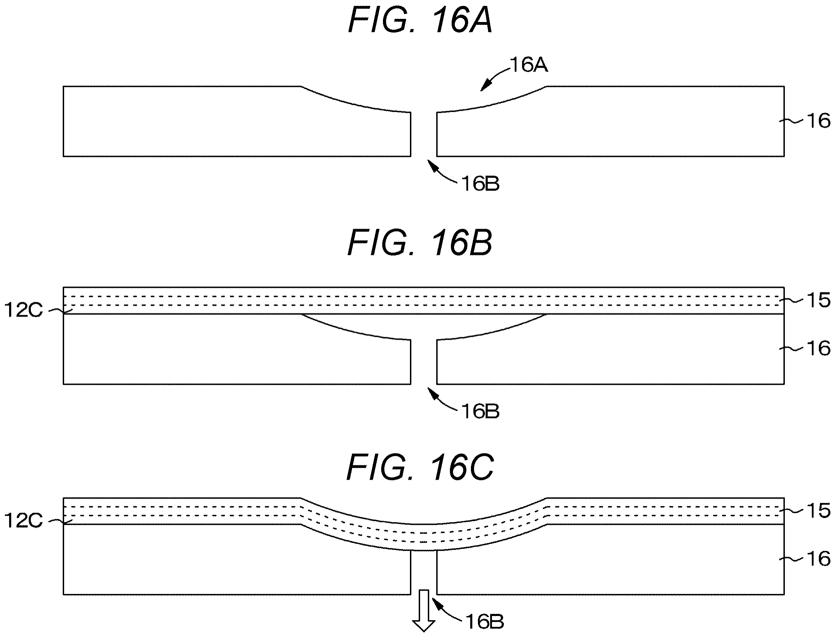

Figs. 16A, 16B, and 16C are schematic diagrams illustrating vacuum forming in the fifth method of manufacturing a bandpass filter. A suction die 16 (mold) is prepared in which a concave portion 16A having a predetermined curvature is formed on one surface, and an opening 16B is formed in the vicinity of the center of the concave portion 16A and passes through to the other surface side (see Fig. 16A). Next, on the surface of the suction die 16 on which the concave portion 16A is formed, the film sheet 15 is placed so that the reflective film may face upward (so that the antireflection film and the suction die may face each other) (see Fig. 16B). Thereafter, air in the concave portion 16A is sucked from the other surface of the suction die 16 through the opening 16B, and the film sheet 15 is plastically deformed (see Fig. 16C). Next, by removing the film sheet 15 from the suction die 16, the film sheet 15 in which a concave portion having the predetermined curvature is formed can be obtained.

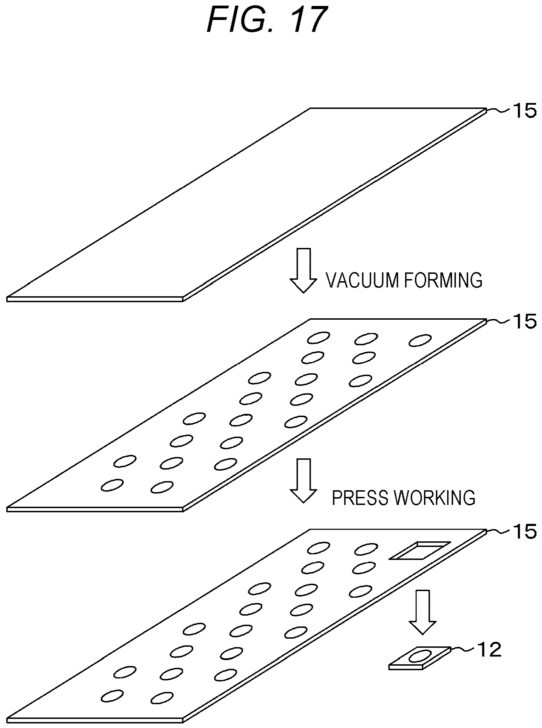

Fig. 17 is a schematic diagram illustrating press working in the fifth method of manufacturing a bandpass filter. The film sheet 15 is subjected to vacuum forming by the method illustrated in Figs. 16A, 16B, and 16C to form a plurality of concave portions on the film sheet 15. Thereafter, the bandpass filter 12 can be obtained by singulation into a predetermined shape including a concave portion by press working.

By using the fifth manufacturing method, a bandpass filter layer can be vapor-deposited on the planar film sheet, so that the bandpass filter layer can be vapor-deposited uniformly and the manufacturing cost can be reduced.

The light receiving unit 20 and the optical member 10 can also be configured as an integrated light receiving module. A method of manufacturing a light receiving module and the like will be described below.

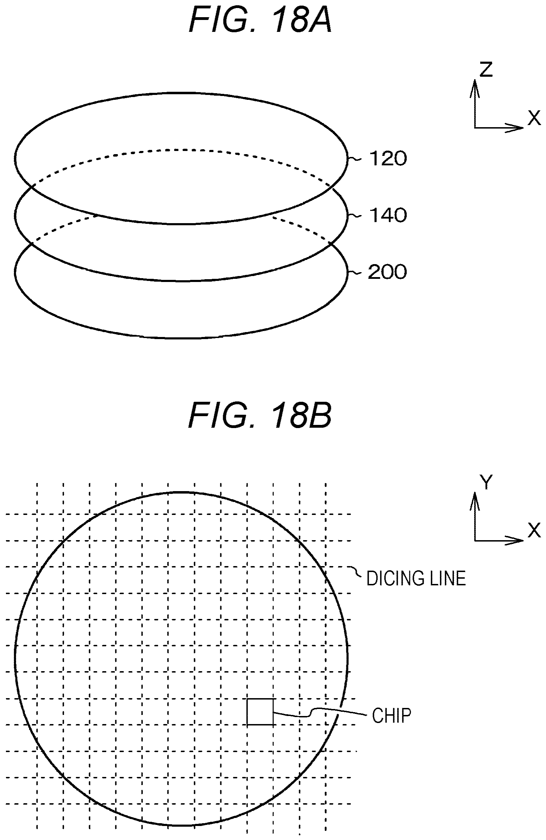

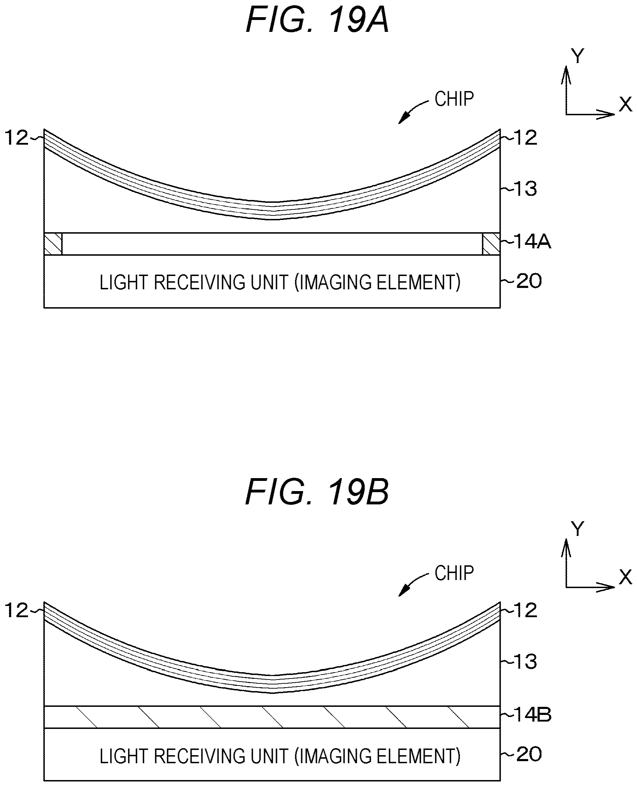

Figs. 18A and 18B are schematic diagrams illustrating a method of manufacturing a light receiving module. Figs. 19A and 19B are schematic diagrams illustrating a structure of a light receiving module.

A semiconductor wafer 200 on which a plurality of imaging elements is formed, a wafer-like frame 140 in which an opening corresponding to a light receiving surface is formed, and a wafer 120 on which a plurality of bandpass filters is formed are stacked (see Fig. 18A), and then, diced and singulated into chips having a predetermined shape (see Fig. 18B). Fig. 19A illustrates a cross section of a singulated chip. Reference numeral 14A indicates a frame. In this configuration, a cavity exists between the base material 13 and the light receiving unit 20.

In some cases, the frame 140 having the opening may be replaced with an adhesive member having no opening in the configuration. Fig. 19B illustrates a cross section of a singulated chip having such a configuration. Reference numeral 14B indicates an adhesive member. In this configuration, no cavity exists between the base material 13 and the light receiving unit 20.

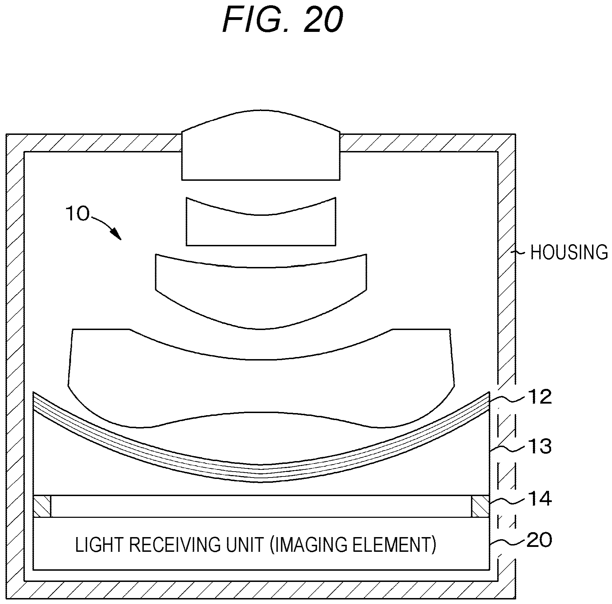

Fig. 20 illustrates an example of a light receiving module further including a lens. In this configuration, a chip manufactured as described above and a lens are incorporated in a housing.

The method of manufacturing a light receiving module and the like have been described above.

As described above, the light receiving unit 20, the analog-to-digital conversion unit 30, the arithmetic processing unit 40, the controller 50, and the light source driving unit 60 illustrated in Fig. 1 may be configured as a single chip, or may be configured as a plurality of chips in accordance with their functions. Figs. 21A, 21B, and 21C are schematic diagrams illustrating a configuration of a semiconductor device used in the distance measuring system.

Subsequently, acquisition of distance information will be described. In the distance measuring system 1 illustrated in Fig. 1, the arithmetic processing unit 40 may have a configuration in which distance information is obtained on the basis of a time of flight of light reflected from a target object, or may have a configuration in which infrared light is emitted in a predetermined pattern to a target object and the arithmetic processing unit 40 obtains distance information on the basis of a pattern of light reflected from the target object. These will be described below as various modified examples.

### [First modified example]

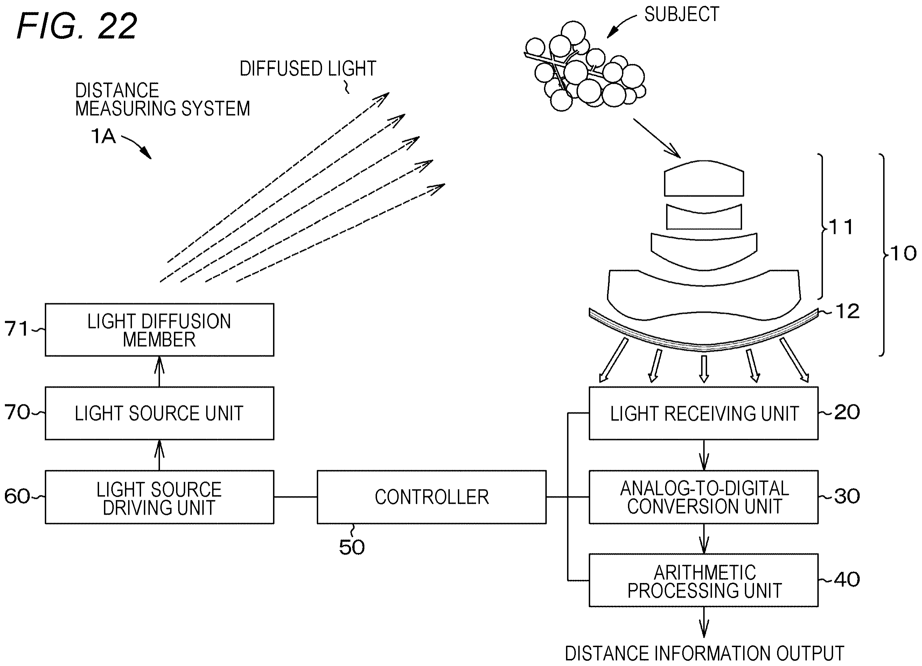

Fig. 22 illustrates a configuration in which distance information is obtained on the basis of the time of flight of reflected light. In a distance measuring system 1A, a light diffusion member 71 is arranged in front of the light source unit 70 to emit diffused light. The light source unit 70 is modulated at a frequency of, for example, several tens of kHz to several hundreds of MHz. Then, distance information can be obtained by detecting a reflected light component in synchronization with the modulation of the light source unit 70.

### [Second modified example]

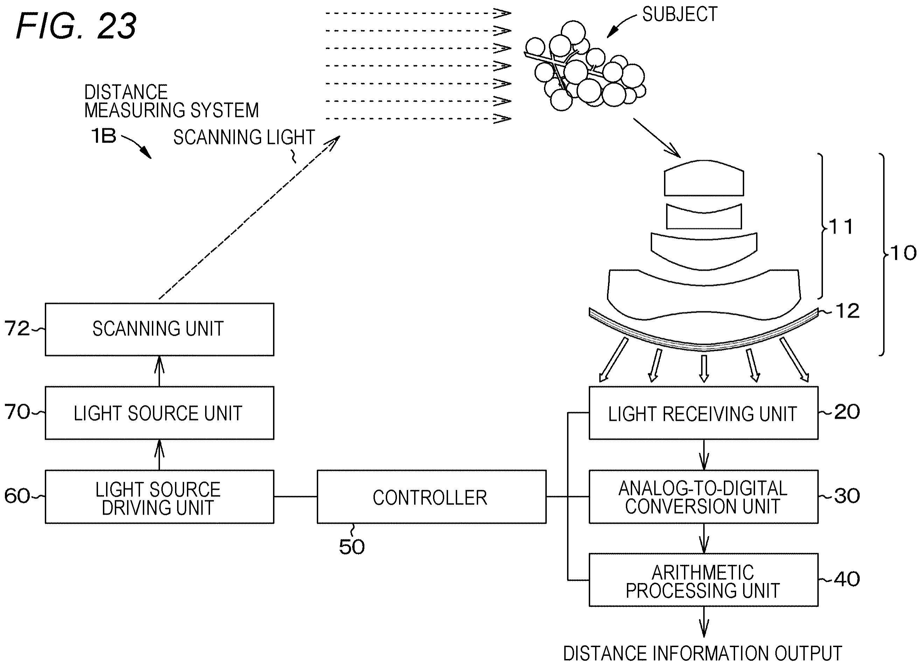

Fig. 23 also illustrates a configuration in which distance information is obtained on the basis of the time of flight of reflected light. In a distance measuring system 1B, a scanning unit 72 causes light from the light source unit 70 to scan. Then, distance information can be obtained by detecting a reflected light component in synchronization with the scanning.

### [Third modified example]

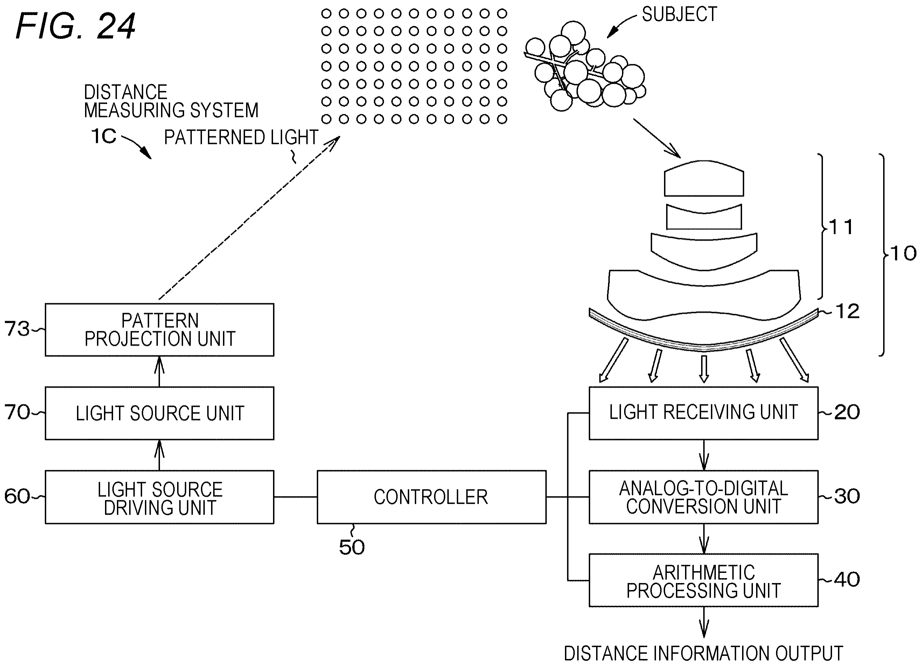

Fig. 24 illustrates a configuration in which infrared light is emitted in a predetermined pattern to a target object, and the arithmetic processing unit 40 obtains distance information on the basis of a pattern of light reflected from the target object. In a distance measuring system 1C, a pattern projection unit 73 causes light from the light source unit 70 to be emitted in a predetermined pattern to a target object. Distance information can be obtained by detecting information regarding spatial distribution of the illuminance pattern or distortion of a pattern image on the target object.

### [Fourth modified example]

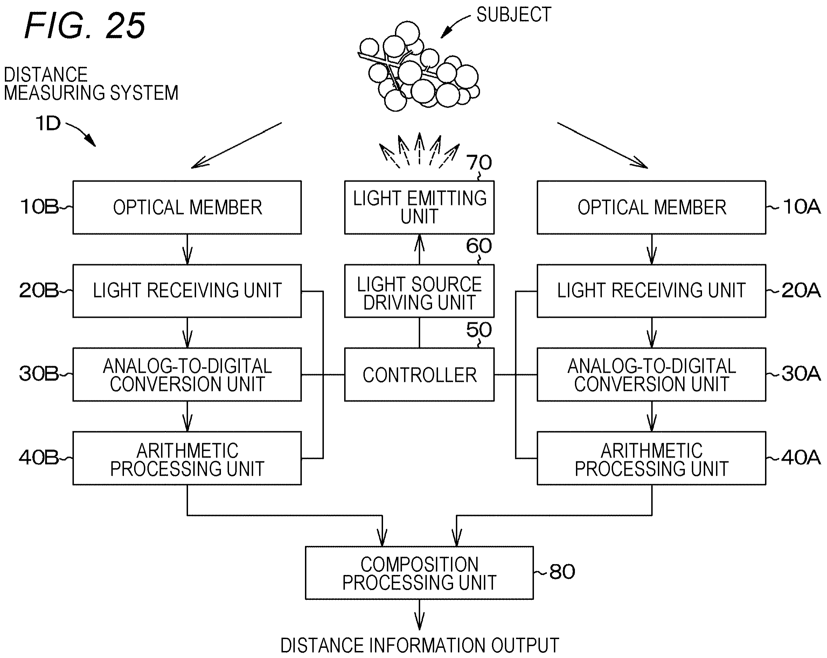

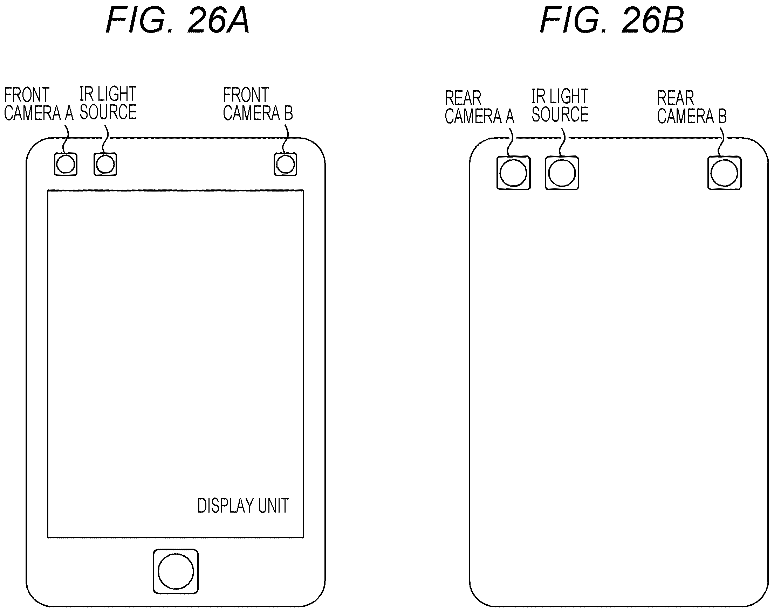

Fig. 25 illustrates a configuration in which stereoscopic information is also obtained by arranging a plurality of light receiving units at a distance from one another. Note that the configuration may be any of the following configurations: a configuration in which diffused light is emitted as in the first modified example, a configuration in which light from the light source scans as in the second modified example, or a configuration in which light is emitted in a predetermined pattern as in the third modified example. Figs. 26A and 26B are schematic diagrams illustrating an example of arrangement of a light receiving unit and a light source unit in a case where they are deployed in portable electronic equipment.

In the first embodiment, the band of the bandpass filter can be narrowed, and the influence of disturbance light can be reduced. Thus, high-quality ranging imaging can be achieved even under external light. Furthermore, a light receiving module having excellent wavelength selectivity can be provided by setting the shape of a bandpass filter in accordance with a lens module.

### [First application example]

The technology according to the present disclosure can be applied to a variety of products. For example, the technology according to the present disclosure may be materialized as a device that is mounted on any type of mobile object such as an automobile, an electric vehicle, a hybrid electric vehicle, a motorcycle, a bicycle, personal mobility, an airplane, a drone, a ship, a robot, a construction machine, or an agricultural machine (tractor).

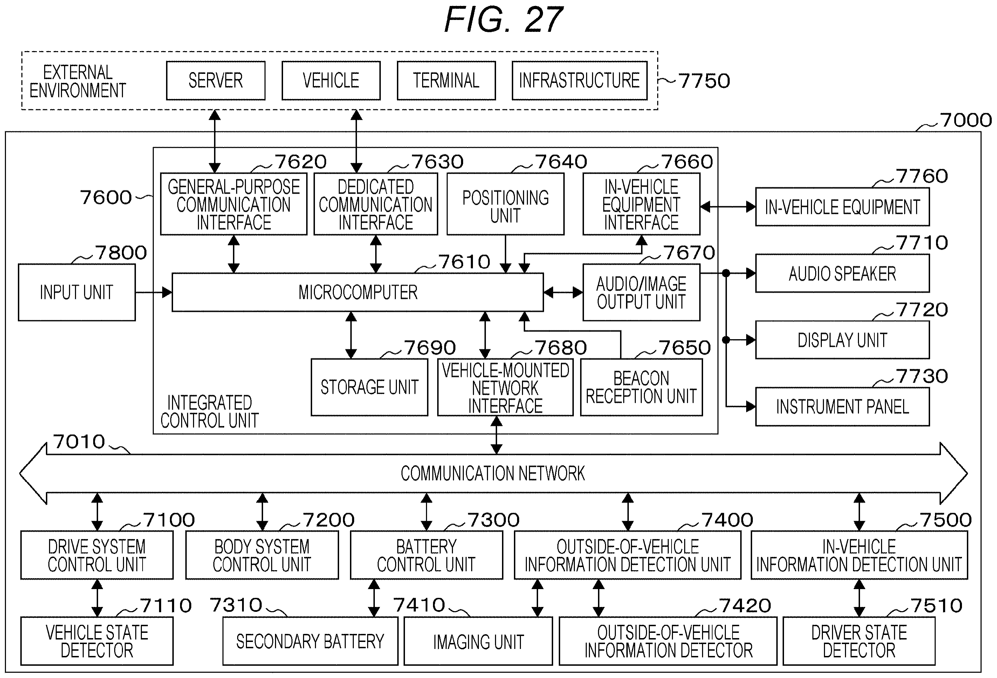

Fig. 27 is a block diagram illustrating a schematic configuration example of a vehicle control system 7000 that is an example of a mobile object control system to which the technology according to the present disclosure can be applied. The vehicle control system 7000 includes a plurality of electronic control units connected via a communication network 7010. In the example illustrated in Fig. 27, the vehicle control system 7000 includes a drive system control unit 7100, a body system control unit 7200, a battery control unit 7300, an outside-of-vehicle information detection unit 7400, an in-vehicle information detection unit 7500, and an integrated control unit 7600. The communication network 7010 connecting the plurality of control units may be, for example, a controller area network (CAN), a local interconnect network (LIN), a local area network (LAN), or a vehicle-mounted communication network that conforms to an optional standard such as FlexRay (registered trademark).

Each control unit includes a microcomputer that performs arithmetic processing in accordance with various programs, a storage unit that stores a program executed by the microcomputer, a parameter used for various computations, or the like, and a drive circuit that drives a device on which various controls are performed. Each control unit includes a network interface for performing communication with another control unit via the communication network 7010, and also includes a communication interface for performing wired or wireless communication with a device, sensor, or the like inside or outside a vehicle. Fig. 27 illustrates a functional configuration of the integrated control unit 7600, which includes a microcomputer 7610, a general-purpose communication interface 7620, a dedicated communication interface 7630, a positioning unit 7640, a beacon reception unit 7650, an in-vehicle equipment interface 7660, an audio/image output unit 7670, a vehicle-mounted network interface 7680, and a storage unit 7690. In a similar manner, other control units also include a microcomputer, a communication interface, a storage unit, and the like.

The drive system control unit 7100 controls operation of devices related to a drive system of the vehicle in accordance with various programs. For example, the drive system control unit 7100 functions as a device for controlling a driving force generation device for generating a driving force of the vehicle such as an internal combustion engine or a driving motor, a driving force transmission mechanism for transmitting the driving force to wheels, a steering mechanism that regulates a steering angle of the vehicle, a braking device that generates a braking force of the vehicle, and the like. The drive system control unit 7100 may have a function as a device for controlling an antilock brake system (ABS), an electronic stability control (ESC), or the like.

The drive system control unit 7100 is connected with a vehicle state detector 7110. The vehicle state detector 7110 includes, for example, at least one of a gyro sensor that detects an angular velocity of shaft rotation of a vehicle body, an acceleration sensor that detects an acceleration of the vehicle, or a sensor for detecting an operation amount of an accelerator pedal, an operation amount of a brake pedal, a steering angle of a steering wheel, an engine speed, a wheel rotation speed, or the like. The drive system control unit 7100 performs arithmetic processing using a signal input from the vehicle state detector 7110, and controls the internal combustion engine, the driving motor, an electric power steering device, a brake device, or the like.

The body system control unit 7200 controls operation of various devices mounted on the vehicle body in accordance with various programs. For example, the body system control unit 7200 functions as a device for controlling a keyless entry system, a smart key system, a power window device, or various lamps such as a head lamp, a back lamp, a brake lamp, a blinker, or a fog lamp. In this case, radio waves transmitted from a portable device that substitutes for a key or signals from various switches can be input to the body system control unit 7200. The body system control unit 7200 receives the input of these radio waves or signals, and controls a door lock device, the power window device, a lamp, and the like of the vehicle.

The battery control unit 7300 controls a secondary battery 7310 that is a power supply source of the driving motor in accordance with various programs. For example, information such as a battery temperature, a battery output voltage, or a battery remaining capacity is input to the battery control unit 7300 from a battery device including the secondary battery 7310. The battery control unit 7300 performs arithmetic processing using these signals, and performs temperature regulation control of the secondary battery 7310 or control of a cooling device or the like included in the battery device.

The outside-of-vehicle information detection unit 7400 detects information outside the vehicle on which the vehicle control system 7000 is mounted. For example, the outside-of-vehicle information detection unit 7400 is connected with at least one of an imaging unit 7410 or an outside-of-vehicle information detector 7420. The imaging unit 7410 includes at least one of a time of flight (ToF) camera, a stereo camera, a monocular camera, an infrared camera, or another camera. The outside-of-vehicle information detector 7420 includes, for example, at least one of an environment sensor for detecting the current weather or climate, or a surrounding information detection sensor for detecting another vehicle, an obstacle, a pedestrian, or the like in the surroundings of the vehicle on which the vehicle control system 7000 is mounted.

The environment sensor may be, for example, at least one of a raindrop sensor that detects rainy weather, a fog sensor that detects fog, a sunshine sensor that detects the degree of sunshine, or a snow sensor that detects snowfall. The surrounding information detection sensor may be at least one of an ultrasonic sensor, a radar device, or a LIDAR ("light detection and ranging" or "laser imaging detection and ranging") device. These imaging unit 7410 and outside-of-vehicle information detector 7420 may each be disposed as an independent sensor or device, or may be disposed as an integrated device including a plurality of sensors or devices.

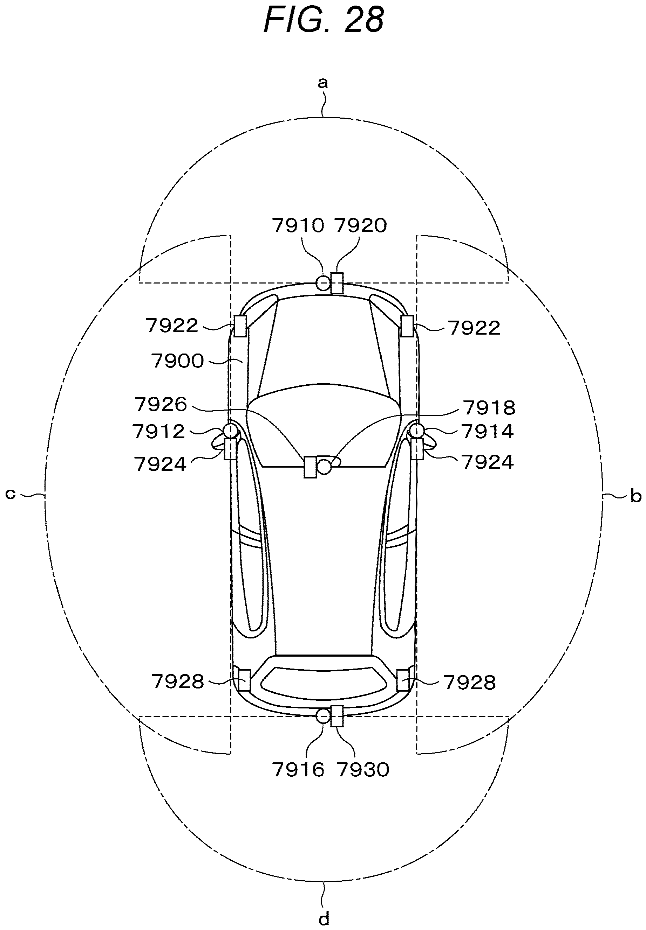

Here, Fig. 28 illustrates an example of installation positions of the imaging unit 7410 and the outside-of-vehicle information detector 7420. Imaging units 7910, 7912, 7914, 7916, and 7918 are provided at, for example, at least one of a front nose, a side mirror, a rear bumper, a back door, or the top of a windshield in a vehicle interior of a vehicle 7900. The imaging unit 7910 disposed at the front nose and the imaging unit 7918 disposed at the top of the windshield in the vehicle interior mainly acquire an image in front of the vehicle 7900. The imaging units 7912 and 7914 disposed at the side mirror mainly acquire images of side views from the vehicle 7900. The imaging unit 7916 disposed at the rear bumper or the back door mainly acquires an image behind the vehicle 7900. The imaging unit 7918 disposed at the top of the windshield in the vehicle interior is mainly used to detect a preceding vehicle, a pedestrian, an obstacle, a traffic light, a traffic sign, a lane, or the like.

Note that Fig. 28 illustrates an example of an imaging range of each of the imaging units 7910, 7912, 7914, and 7916. An imaging range a indicates an imaging range of the imaging unit 7910 provided at the front nose, imaging ranges b and c respectively indicate imaging ranges of the imaging units 7912 and 7914 provided at the side mirrors, and an imaging range d indicates an imaging range of the imaging unit 7916 provided at the rear bumper or the back door. For example, a bird's-eye view image of the vehicle 7900 viewed from above can be obtained by superimposing pieces of image data captured by the imaging units 7910, 7912, 7914, and 7916.

Outside-of-vehicle information detectors 7920, 7922, 7924, 7926, 7928, and 7930 provided at the front, rear, sides, and corners of the vehicle 7900, and the top of the windshield in the vehicle interior may be, for example, ultrasonic sensors or radar devices. The outside-of-vehicle information detectors 7920, 7926, and 7930 provided at the front nose, the rear bumper, the back door, and the top of the windshield in the vehicle interior of the vehicle 7900 may be, for example, LIDAR devices. These outside-of-vehicle information detectors 7920 to 7930 are mainly used to detect a preceding vehicle, a pedestrian, an obstacle, or the like.

Returning to Fig. 27, the description will be continued. The outside-of-vehicle information detection unit 7400 causes the imaging unit 7410 to capture an image of the outside of the vehicle, and receives the captured image data. Furthermore, the outside-of-vehicle information detection unit 7400 receives detection information from the connected outside-of-vehicle information detector 7420. In a case where the outside-of-vehicle information detector 7420 is an ultrasonic sensor, a radar device, or a LIDAR device, the outside-of-vehicle information detection unit 7400 transmits ultrasonic waves, electromagnetic waves, or the like, and receives information from received reflected waves. The outside-of-vehicle information detection unit 7400 may perform object detection processing or distance detection processing of a person, a car, an obstacle, a sign, a character on a road surface, or the like on the basis of the received information. The outside-of-vehicle information detection unit 7400 may perform environment recognition processing for recognizing rainfall, fog, road surface conditions, or the like on the basis of the received information. The outside-of-vehicle information detection unit 7400 may calculate a distance to an object outside the vehicle on the basis of the received information.

Furthermore, the outside-of-vehicle information detection unit 7400 may perform image recognition processing or distance detection processing for recognizing a person, a car, an obstacle, a sign, a character on a road surface, or the like on the basis of the received image data. The outside-of-vehicle information detection unit 7400 may also generate a bird's-eye view image or a panoramic image by performing processing such as distortion correction or positioning on the received image data, and generating a composite image from pieces of image data captured by different imaging units 7410. The outside-of-vehicle information detection unit 7400 may perform viewpoint conversion processing using pieces of image data captured by the different imaging units 7410.

The in-vehicle information detection unit 7500 detects information inside the vehicle. The in-vehicle information detection unit 7500 is connected with, for example, a driver state detector 7510 that detects a state of a driver. The driver state detector 7510 may include a camera that captures an image of the driver, a biological sensor that detects biological information of the driver, a microphone that collects sounds in the vehicle interior, or the like. The biological sensor is provided at, for example, a seat surface, the steering wheel, or the like, and detects biological information of an occupant sitting on a seat or a driver gripping the steering wheel. On the basis of detection information input from the driver state detector 7510, the in-vehicle information detection unit 7500 may calculate the degree of fatigue or concentration of the driver, or determine whether or not the driver has fallen asleep. The in-vehicle information detection unit 7500 may perform processing such as noise canceling processing on signals of collected sounds.

The integrated control unit 7600 controls overall operation in the vehicle control system 7000 in accordance with various programs. The integrated control unit 7600 is connected with an input unit 7800. The input unit 7800 includes a device that can be used by an occupant to perform an input operation, for example, a touch panel, a button, a microphone, a switch, a lever, or the like. Data obtained by speech recognition of speech input via the microphone may be input to the integrated control unit 7600. The input unit 7800 may be, for example, a remote control device using infrared rays or other radio waves, or may be externally connected equipment such as a mobile phone or a personal digital assistant (PDA) that can be used to operate the vehicle control system 7000. The input unit 7800 may be, for example, a camera, in which case an occupant can input information by gesture. Alternatively, data to be input may be obtained by detecting a movement of a wearable appliance worn by an occupant. Moreover, the input unit 7800 may include, for example, an input control circuit that generates an input signal on the basis of information input by an occupant or the like using the input unit 7800 described above, and outputs the input signal to the integrated control unit 7600. By operating the input unit 7800, an occupant or the like inputs various types of data to the vehicle control system 7000 or gives an instruction on a processing operation.

The storage unit 7690 may include a read only memory (ROM) for storing various programs executed by a microcomputer, and a random access memory (RAM) for storing various parameters, computation results, sensor values, or the like. Furthermore, the storage unit 7690 may include a magnetic storage device such as a hard disc drive (HDD), a semiconductor storage device, an optical storage device, a magneto-optical storage device, or the like.

The general-purpose communication interface 7620 is a versatile communication interface that mediates communication with a variety of types of equipment existing in an external environment 7750. The general-purpose communication interface 7620 may implement a cellular communication protocol such as global system of mobile communications (GSM) (registered trademark), WiMAX, long term evolution (LTE), or LTE-advanced (LTE-A), or another wireless communication protocol such as wireless LAN (also referred to as Wi-Fi (registered trademark)) or Bluetooth (registered trademark). The general-purpose communication interface 7620 may be connected to equipment (for example, an application server or a control server) existing on an external network (for example, the Internet, a cloud network, or an operator-specific network) via, for example, a base station or an access point. Furthermore, the general-purpose communication interface 7620 may be connected to, for example, using peer-to-peer (P2P) technology, a terminal existing near the vehicle (for example, a terminal of a driver, pedestrian, or store, or a machine type communication (MTC) terminal).

The dedicated communication interface 7630 is a communication interface that supports a communication protocol designed for use in a vehicle. The dedicated communication interface 7630 may implement, for example, a standard protocol such as wireless access in vehicle environment (WAVE), which is a combination of lower-layer IEEE802.11p and upper-layer IEEE1609, dedicated short range communications (DSRC), or a cellular communication protocol. The dedicated communication interface 7630 typically performs V2X communication, which is a concept that includes at least one of vehicle to vehicle communication, vehicle to infrastructure communication, vehicle to home communication, or vehicle to pedestrian communication.

For example, the positioning unit 7640 receives a global navigation satellite system (GNSS) signal from a GNSS satellite (for example, a global positioning system (GPS) signal from a GPS satellite), executes positioning, and generates position information including the latitude, longitude, and altitude of the vehicle. Note that the positioning unit 7640 may specify a current position by exchanging signals with a wireless access point, or may acquire position information from a terminal such as a mobile phone, a PHS, or a smartphone having a positioning function.

For example, the beacon reception unit 7650 receives radio waves or electromagnetic waves transmitted from a wireless station or the like installed on a road to acquire information such as a current position, traffic congestion, suspension of traffic, or required time. Note that the function of the beacon reception unit 7650 may be included in the dedicated communication interface 7630 described above.

The in-vehicle equipment interface 7660 is a communication interface that mediates connections between the microcomputer 7610 and a variety of types of in-vehicle equipment 7760 existing inside the vehicle. The in-vehicle equipment interface 7660 may establish a wireless connection using a wireless communication protocol such as wireless LAN, Bluetooth (registered trademark), near field communication (NFC), or wireless USB (WUSB). Furthermore, the in-vehicle equipment interface 7660 may establish a wired connection such as universal serial bus (USB), high-definition multimedia interface (HDMI) (registered trademark), or mobile high-definition link (MHL) via a connection terminal (not illustrated) (and, if necessary, a cable). The in-vehicle equipment 7760 may include, for example, at least one of mobile equipment or wearable equipment possessed by an occupant, or information equipment carried in or attached to the vehicle. Furthermore, the in-vehicle equipment 7760 may include a navigation device that searches for a route to an optional destination. The in-vehicle equipment interface 7660 exchanges control signals or data signals with the in-vehicle equipment 7760.

The vehicle-mounted network interface 7680 is an interface that mediates communication between the microcomputer 7610 and the communication network 7010. The vehicle-mounted network interface 7680 transmits and receives signals and the like on the basis of a predetermined protocol supported by the communication network 7010.

On the basis of information acquired via at least one of the general-purpose communication interface 7620, the dedicated communication interface 7630, the positioning unit 7640, the beacon reception unit 7650, the in-vehicle equipment interface 7660, or the vehicle-mounted network interface 7680, the microcomputer 7610 of the integrated control unit 7600 controls the vehicle control system 7000 in accordance with various programs. For example, the microcomputer 7610 may compute a control target value for the driving force generation device, the steering mechanism, or the braking device on the basis of information acquired from the inside and outside of the vehicle, and output a control command to the drive system control unit 7100. For example, the microcomputer 7610 may perform cooperative control for the purpose of implementing functions of an advanced driver assistance system (ADAS) including collision avoidance or shock mitigation of the vehicle, follow-up traveling based on an inter-vehicle distance, vehicle speed maintaining traveling, vehicle collision warning, vehicle lane departure warning, or the like. Furthermore, the microcomputer 7610 may perform cooperative control for the purpose of automatic operation, that is, autonomous driving without the driver's operation, or the like by controlling the driving force generation device, the steering mechanism, the braking device, or the like on the basis of information acquired from the surroundings of the vehicle.

The microcomputer 7610 may generate information regarding a three-dimensional distance between the vehicle and an object such as a structure or a person in the periphery of the vehicle and create local map information including information in the periphery of the current position of the vehicle on the basis of information acquired via at least one of the general-purpose communication interface 7620, the dedicated communication interface 7630, the positioning unit 7640, the beacon reception unit 7650, the in-vehicle equipment interface 7660, or the vehicle-mounted network interface 7680. Furthermore, the microcomputer 7610 may predict a danger such as a collision of the vehicle, approaching a pedestrian or the like, or entering a closed road on the basis of the acquired information, and generate a warning signal. The warning signal may be, for example, a signal for generating a warning sound or lighting a warning lamp.

The audio/image output unit 7670 transmits at least one of an audio output signal or an image output signal to an output device capable of visually or aurally notifying an occupant in the vehicle or the outside of the vehicle of information. In the example of Fig. 27, an audio speaker 7710, a display unit 7720, and an instrument panel 7730 are illustrated as the output device. The display unit 7720 may include, for example, at least one of an on-board display or a head-up display. The display unit 7720 may have an augmented reality (AR) display function. Other than these devices, the output device may be another device such as a headphone, a wearable device such as a glasses-type display worn by an occupant, a projector, or a lamp. In a case where the output device is a display device, the display device visually displays, in a variety of forms such as text, images, tables, or graphs, results obtained from various types of processing performed by the microcomputer 7610 or information received from another control unit. Furthermore, in a case where the output device is an audio output device, the audio output device converts an audio signal including reproduced audio data, acoustic data, or the like into an analog signal and aurally outputs the analog signal.

Note that, in the example illustrated in Fig. 27, at least two control units connected via the communication network 7010 may be integrated as one control unit. Alternatively, each control unit may include a plurality of control units. Moreover, the vehicle control system 7000 may include another control unit (not illustrated). Furthermore, in the above description, some or all of the functions performed by one of the control units may be provided to another control unit. That is, as long as information is transmitted and received via the communication network 7010, predetermined arithmetic processing may be performed by any of the control units. Similarly, a sensor or device connected to any control unit may be connected to another control unit, and a plurality of control units may transmit and receive detection information to and from each other via the communication network 7010.

The technology according to the present disclosure may be applied to, for example, an imaging unit of an outside-of-vehicle information detection unit among the configurations described above.

### [Second application example]

The technology according to the present disclosure can be applied to a variety of products. For example, the technology according to the present disclosure may be applied to an endoscopic surgery system.

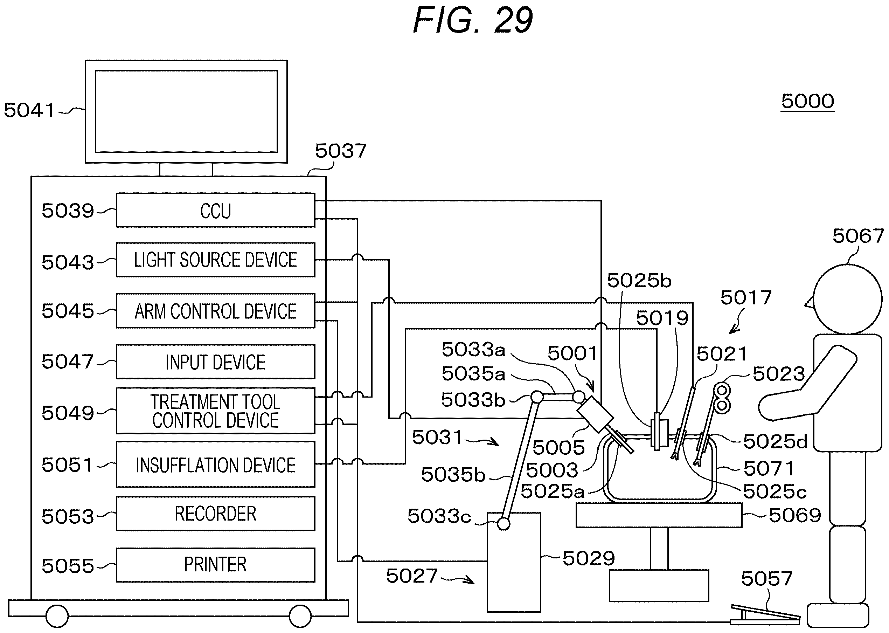

Fig. 29 is a diagram illustrating an example of a schematic configuration of an endoscopic surgery system 5000 to which the technology according to the present disclosure may be applied. Fig. 29 illustrates a situation in which an operator (doctor) 5067 is performing surgery on a patient 5071 on a patient bed 5069 using the endoscopic surgery system 5000. As illustrated, the endoscopic surgery system 5000 includes an endoscope 5001, other surgical tools 5017, a support arm device 5027 that supports the endoscope 5001, and a cart 5037 on which various devices for endoscopic surgery are mounted.

In endoscopic surgery, an abdominal wall is pierced with a plurality of tubular hole-opening instruments called trocars 5025a to 5025d, instead of cutting and opening the abdominal wall. Then, a lens barrel 5003 of the endoscope 5001 and the other surgical tools 5017 are inserted into a body cavity of the patient 5071 through the trocars 5025a to 5025d. In the illustrated example, an insufflation tube 5019, an energy treatment tool 5021, and forceps 5023 are inserted into the body cavity of the patient 5071 as the other surgical tools 5017. Furthermore, the energy treatment tool 5021 is used to perform incision and exfoliation of tissue, sealing of a blood vessel, or the like by using a high-frequency current or ultrasonic vibration. However, the illustrated surgical tools 5017 are merely an example, and various surgical tools generally used in endoscopic surgery, such as tweezers, a retractor, and the like, may be used as the surgical tools 5017.

An image of a surgical site in the body cavity of the patient 5071 captured by the endoscope 5001 is displayed on a display device 5041. The operator 5067 performs a procedure such as excision of an affected part, for example, using the energy treatment tool 5021 or the forceps 5023 while viewing the image of the surgical site displayed on the display device 5041 in real time. Note that, although not illustrated, the insufflation tube 5019, the energy treatment tool 5021, and the forceps 5023 are supported by the operator 5067, an assistant, or the like during the surgery.

### (Support arm device)

The support arm device 5027 includes an arm 5031 extending from a base portion 5029. In the illustrated example, the arm 5031 includes joints 5033a, 5033b, and 5033c, and links 5035a and 5035b, and is driven by control of an arm control device 5045. The arm 5031 supports the endoscope 5001 so as to control its position and orientation. With this arrangement, the position of the endoscope 5001 can be stably fixed.

### (Endoscope)

The endoscope 5001 includes the lens barrel 5003 whose predetermined length from an end is inserted into the body cavity of the patient 5071, and a camera head 5005 connected to a proximal end of the lens barrel 5003. In the illustrated example, the endoscope 5001 configured as a so-called rigid endoscope having the lens barrel 5003 that is rigid is illustrated. Alternatively, the endoscope 5001 may be configured as a so-called flexible endoscope having the lens barrel 5003 that is flexible.

The lens barrel 5003 is provided with, at the end thereof, an opening portion in which an objective lens is fitted. The endoscope 5001 is connected with a light source device 5043. Light generated by the light source device 5043 is guided to the end of the lens barrel 5003 by a light guide extending inside the lens barrel, and is emitted through the objective lens toward an observation target in the body cavity of the patient 5071. Note that the endoscope 5001 may be a forward-viewing endoscope, an oblique-viewing endoscope, or a side-viewing endoscope.

The camera head 5005 is provided with an optical system and an imaging element inside thereof, and light reflected from the observation target (observation light) is focused on the imaging element by the optical system. The imaging element photoelectrically converts the observation light to generate an electric signal corresponding to the observation light, that is, an image signal corresponding to an observation image. The image signal is transmitted to a camera control unit (CCU) 5039 as raw data. Note that the camera head 5005 has a function of adjusting a magnification and a focal length by appropriately driving the optical system.

Note that the camera head 5005 may be provided with a plurality of imaging elements in order to support, for example, stereoscopic viewing (3D display) and the like. In this case, the lens barrel 5003 is provided with a plurality of relay optical systems inside thereof to guide observation light to every one of the plurality of imaging elements.

### (Various devices mounted on cart)

The CCU 5039 is constituted by a central processing unit (CPU), a graphics processing unit (GPU), and the like, and integrally controls operations of the endoscope 5001 and the display device 5041. Specifically, the CCU 5039 performs, on an image signal received from the camera head 5005, various types of image processing for displaying an image based on the image signal, such as development processing (demosaic processing), for example. The CCU 5039 provides the display device 5041 with the image signal on which image processing has been performed. Furthermore, the CCU 5039 transmits a control signal to the camera head 5005 to control its driving. The control signal may contain information regarding imaging conditions such as the magnification and the focal length.

The CCU 5039 controls the display device 5041 to display an image based on the image signal on which image processing has been performed by the CCU 5039. In a case where, for example, the endoscope 5001 supports imaging with a high resolution such as 4K (3840 horizontal pixels x 2160 vertical pixels) or 8K (7680 horizontal pixels x 4320 vertical pixels), and/or in a case where the endoscope 5001 supports 3D display, a display device supporting high-resolution display and/or 3D display can be used accordingly as the display device 5041. In a case where imaging with a high resolution such as 4K or 8K is supported, a display device having a size of 55 inches or more can be used as the display device 5041 to provide more immersive feeling. Furthermore, a plurality of display devices 5041 having different resolutions and sizes may be provided depending on the intended use.

The light source device 5043 includes a light source such as a light emitting diode (LED), for example, and supplies the endoscope 5001 with emitted light at the time of imaging a surgical site.

The arm control device 5045 is constituted by a processor such as a CPU, for example, and operates in accordance with a predetermined program to control driving of the arm 5031 of the support arm device 5027 in accordance with a predetermined control method.

An input device 5047 is an input interface to the endoscopic surgery system 5000. A user can input various types of information and input instructions to the endoscopic surgery system 5000 via the input device 5047. For example, the user inputs, via the input device 5047, various types of information related to surgery, such as physical information of a patient and information regarding a surgical procedure. Furthermore, for example, the user may input, via the input device 5047, an instruction to drive the arm 5031, an instruction to change imaging conditions (the type of emitted light, the magnification and focal length, and the like) of the endoscope 5001, an instruction to drive the energy treatment tool 5021, and the like.

The type of the input device 5047 is not limited, and various known input devices may be used as the input device 5047. As the input device 5047, for example, a mouse, a keyboard, a touch panel, a switch, a foot switch 5057, and/or a lever can be applied. In a case where a touch panel is used as the input device 5047, the touch panel may be provided on a display surface of the display device 5041.

Alternatively, the input device 5047 is a device worn by a user, such as a glasses-type wearable device or a head mounted display (HMD), for example, and various inputs are performed in accordance with a user's gesture or line-of-sight detected by these devices. Furthermore, the input device 5047 includes a camera capable of detecting a movement of a user, and various inputs are performed in accordance with a user's gesture or line-of-sight detected from a video captured by the camera. Moreover, the input device 5047 includes a microphone capable of collecting a user's voice, and various inputs are performed by speech via the microphone. As described above, since the input device 5047 has a configuration in which various types of information can be input in a non-contact manner, in particular, a user belonging to a clean area (for example, the operator 5067) can operate equipment belonging to an unclean area in a non-contact manner. Furthermore, the user can operate the equipment while holding a surgical tool in hand, and this improves convenience of the user.

A treatment tool control device 5049 controls driving of the energy treatment tool 5021 for cauterization or incision of tissue, sealing of a blood vessel, or the like. In order to inflate a body cavity of the patient 5071 for the purpose of securing a field of view of the endoscope 5001 and securing a working space for the operator, an insufflation device 5051 sends gas through the insufflation tube 5019 into the body cavity. A recorder 5053 is a device that can record various types of information related to surgery. A printer 5055 is a device that can print various types of information related to surgery in various formats such as text, images, or graphs.

A particularly characteristic configuration of the endoscopic surgery system 5000 will be described below in more detail.

### (Support arm device)

The support arm device 5027 includes the base portion 5029 as a base, and the arm 5031 extending from the base portion 5029. In the illustrated example, the arm 5031 includes the plurality of joints 5033a, 5033b, and 5033c, and the plurality of links 5035a and 5035b connected by the joint 5033b. However, Fig. 29 illustrates a configuration of the arm 5031 in a simplified manner for ease. In practice, the shapes, the numbers, and the arrangement of the joints 5033a to 5033c and the links 5035a and 5035b, the directions of rotation axes of the joints 5033a to 5033c, and the like can be appropriately set so that the arm 5031 has a desired degree of freedom. For example, the arm 5031 may suitably have a configuration that enables six or more degrees of freedom. With this arrangement, the endoscope 5001 can be freely moved within a movable range of the arm 5031, and the lens barrel 5003 of the endoscope 5001 can be inserted into the body cavity of the patient 5071 from a desired direction.

The joints 5033a to 5033c are provided with actuators, and the joints 5033a to 5033c have a configuration that enables rotation about a predetermined rotation axis by driving of the actuators. The arm control device 5045 controls the driving of the actuators, thereby controlling a rotation angle of each of the joints 5033a to 5033c, and controlling the driving of the arm 5031. With this arrangement, the position and orientation of the endoscope 5001 can be controlled. At this time, the arm control device 5045 can control the driving of the arm 5031 by various known control methods such as force control or position control.

For example, the position and orientation of the endoscope 5001 may be controlled by the operator 5067 performing an appropriate operation input via the input device 5047 (including the foot switch 5057), thereby causing the arm control device 5045 to appropriately control the driving of the arm 5031 in accordance with the operation input. With this control, the endoscope 5001 at an end of the arm 5031 can be moved from an optional position to an optional position, and then fixedly supported at the position after the movement. Note that the arm 5031 may be operated by a so-called master-slave method. In this case, the arm 5031 can be remotely controlled by a user via the input device 5047 installed at a location away from an operating room.

Furthermore, in a case where the force control is applied, so-called power assist control may be performed in which the arm control device 5045 receives an external force from a user and drives the actuators of the corresponding joints 5033a to 5033c so that the arm 5031 moves smoothly in accordance with the external force. With this arrangement, when the user moves the arm 5031 while directly touching the arm 5031, the arm 5031 can be moved with a relatively light force. Thus, the endoscope 5001 can be moved more intuitively and with a simpler operation, and this improves convenience of the user.

Here, in general, the endoscope 5001 has been supported by a doctor called an endoscopist during endoscopic surgery. On the other hand, by using the support arm device 5027, the position of the endoscope 5001 can be fixed more reliably without manual operation. This makes it possible to stably obtain an image of a surgical site and smoothly perform surgery.

Note that the arm control device 5045 is not necessarily provided at the cart 5037. Furthermore, the arm control device 5045 is not necessarily one device. For example, the arm control device 5045 may be provided one for each of the joints 5033a to 5033c of the arm 5031 of the support arm device 5027, and a plurality of the arm control devices 5045 may cooperate with one another to control the driving of the arm 5031.

### (Light source device)

The light source device 5043 supplies the endoscope 5001 with emitted light at the time of imaging a surgical site. The light source device 5043 is constituted by a white light source including, for example, an LED, a laser light source, or a combination thereof. At this time, in a case where the white light source includes a combination of RGB laser light sources, an output intensity and output timing of each color (each wavelength) can be controlled with high precision, and this enables white balance adjustment of a captured image at the light source device 5043. Furthermore, in this case, an image for each of **R, G,** and B can be captured in a time-division manner by emitting laser light from each of the RGB laser light sources to an observation target in a time-division manner, and controlling driving of the imaging element of the camera head 5005 in synchronization with the emission timing. According to this method, a color image can be obtained without providing a color filter in the imaging element.

Furthermore, driving of the light source device 5043 may be controlled so that the intensity of light to be output may change at a predetermined time interval. By controlling the driving of the imaging element of the camera head 5005 in synchronization with the timing of the change in the light intensity, acquiring images in a time-division manner, and generating a composite image from the images, a high dynamic range image without so-called blocked up shadows or blown out highlights can be generated.

Furthermore, the light source device 5043 may have a configuration in which light can be supplied in a predetermined wavelength band that can be used for special light observation. In special light observation, for example, by utilizing wavelength dependence of light absorption in body tissue, so-called narrow band imaging is performed in which a predetermined tissue such as a blood vessel in a mucosal surface layer is imaged with high contrast by emitting light in a band narrower than that of light emitted during normal observation (that is, white light). Alternatively, in special light observation, fluorescence observation may be performed in which an image is obtained by fluorescence generated by emitting excitation light. In fluorescence observation, for example, excitation light is emitted to body tissue and fluorescence from the body tissue is observed (autofluorescence observation), or a fluorescent image is obtained by locally injecting a reagent such as indocyanine green (ICG) into body tissue and emitting excitation light corresponding to a fluorescence wavelength of the reagent to the body tissue. The light source device 5043 may have a configuration in which narrow-band light and/or excitation light that can be used for such special light observation can be supplied.

### (Camera head and CCU)

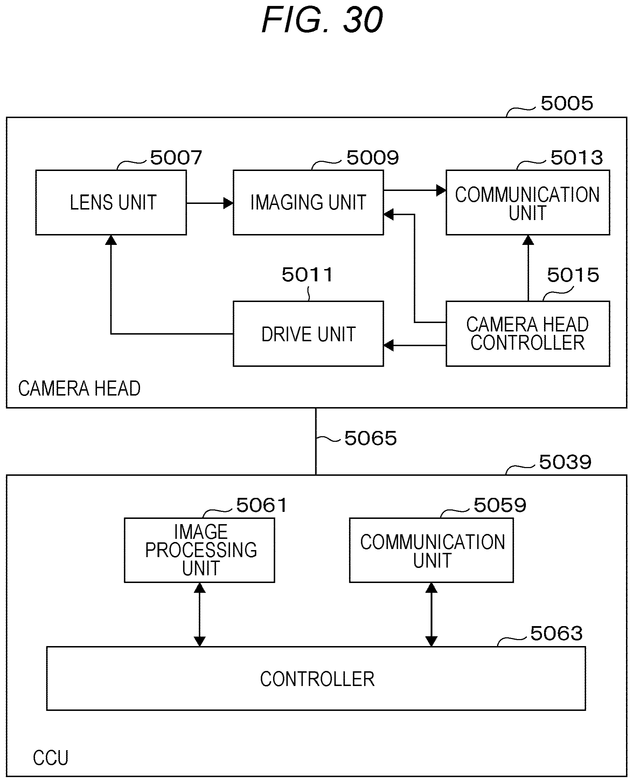

Functions of the camera head 5005 of the endoscope 5001 and the CCU 5039 will be described in more detail with reference to Fig. 30. Fig. 30 is a block diagram illustrating an example of a functional configuration of the camera head 5005 and the CCU 5039 illustrated in Fig. 29.

Referring to Fig. 30, the camera head 5005 has functions including a lens unit 5007, an imaging unit 5009, a driving unit 5011, a communication unit 5013, and a camera head controller 5015. Furthermore, the CCU 5039 has functions including a communication unit 5059, an image processing unit 5061, and a controller 5063. The camera head 5005 and the CCU 5039 are connected by a transmission cable 5065 to allow two-way communication.

First, the functional configuration of the camera head 5005 will be described. The lens unit 5007 is an optical system provided at a connection with the lens barrel 5003. Observation light taken in from the end of the lens barrel 5003 is guided to the camera head 5005 and is incident on the lens unit 5007. The lens unit 5007 is constituted by a combination of a plurality of lenses including a zoom lens and a focus lens. Optical characteristics of the lens unit 5007 are adjusted so that observation light may be focused on a light receiving surface of an imaging element of the imaging unit 5009. Furthermore, the zoom lens and the focus lens have a configuration in which their positions can be moved on an optical axis for adjustment of a magnification and a focus of a captured image.

The imaging unit 5009 is constituted by the imaging element, and is arranged at a stage subsequent to the lens unit 5007. Observation light that has passed through the lens unit 5007 is focused on the light receiving surface of the imaging element, and an image signal corresponding to an observation image is generated by photoelectric conversion. The image signal generated by the imaging unit 5009 is provided to the communication unit 5013.

As the imaging element included in the imaging unit 5009, for example, a complementary metal oxide semiconductor (CMOS) type image sensor that has a Bayer array and can capture color images is used. Note that, as the imaging element, an imaging element capable of capturing a high-resolution image of, for example, 4K or more may be used. An image of a surgical site can be obtained with a high resolution, and this allows the operator 5067 to grasp the state of the surgical site in more detail, and proceed with surgery more smoothly.

Furthermore, the imaging element included in the imaging unit 5009 has a configuration including a pair of imaging elements, one for acquiring a right-eye image signal and the other for acquiring a left-eye image signal supporting 3D display. The 3D display allows the operator 5067 to grasp the depth of living tissue in the surgical site more accurately. Note that, in a case where the imaging unit 5009 has a multiplate type configuration, a plurality of the lens units 5007 is provided to support each of the imaging elements.

Furthermore, the imaging unit 5009 is not necessarily provided in the camera head 5005. For example, the imaging unit 5009 may be provided inside the lens barrel 5003 just behind the objective lens.

The driving unit 5011 is constituted by an actuator, and the camera head controller 5015 controls the zoom lens and the focus lens of the lens unit 5007 to move by a predetermined distance along the optical axis. With this arrangement, the magnification and the focus of an image captured by the imaging unit 5009 can be appropriately adjusted.

The communication unit 5013 is constituted by a communication device for transmitting and receiving various types of information to and from the CCU 5039. The communication unit 5013 transmits an image signal obtained from the imaging unit 5009 as raw data to the CCU 5039 via the transmission cable 5065. At this time, it is preferable that the image signal be transmitted by optical communication in order to display a captured image of a surgical site with a low latency. This is because, during surgery, the operator 5067 performs surgery while observing the state of an affected part from a captured image, and it is required that a moving image of the surgical site be displayed in real time as much as possible for safer and more reliable surgery. In a case where optical communication is performed, the communication unit 5013 is provided with a photoelectric conversion module that converts an electric signal into an optical signal. An image signal is converted into an optical signal by the photoelectric conversion module, and then transmitted to the CCU 5039 via the transmission cable 5065.

Furthermore, the communication unit 5013 receives a control signal for controlling driving of the camera head 5005 from the CCU 5039. The control signal contains, for example, information for specifying a frame rate of a captured image, information for specifying an exposure value at the time of imaging, and/or information for specifying a magnification and focus of the captured image, information regarding imaging conditions, and the like. The communication unit 5013 provides the received control signal to the camera head controller 5015. Note that the control signal from the CCU 5039 may also be transmitted by optical communication. In this case, the communication unit 5013 is provided with a photoelectric conversion module that converts an optical signal into an electric signal. The control signal is converted into an electric signal by the photoelectric conversion module, and then provided to the camera head controller 5015.

Note that the above-described imaging conditions such as the frame rate, the exposure value, the magnification, and the focus are automatically set by the controller 5063 of the CCU 5039 on the basis of an acquired image signal. That is, the endoscope 5001 has a so-called auto exposure (AE) function, an auto focus (AF) function, and an auto white balance (AWB) function.

The camera head controller 5015 controls the driving of the camera head 5005 on the basis of the control signal from the CCU 5039 received via the communication unit 5013. For example, the camera head controller 5015 controls driving of the imaging element of the imaging unit 5009 on the basis of information for specifying a frame rate of a captured image and/or information for specifying exposure at the time of imaging. Furthermore, for example, the camera head controller 5015 appropriately moves the zoom lens and the focus lens of the lens unit 5007 via the driving unit 5011 on the basis of information for specifying a magnification and a focus of a captured image. The camera head controller 5015 may further include a function of storing information for recognizing the lens barrel 5003 and the camera head 5005.

Note that, by arranging the configurations of the lens unit 5007, the imaging unit 5009, and the like in a hermetically sealed structure having high airtightness and waterproofness, the camera head 5005 can have resistance to autoclave sterilization.

Next, the functional configuration of the CCU 5039 will be described. The communication unit 5059 is constituted by a communication device for transmitting and receiving various types of information to and from the camera head 5005. The communication unit 5059 receives an image signal transmitted from the camera head 5005 via the transmission cable 5065. At this time, as described above, the image signal can be suitably transmitted by optical communication. In this case, to support optical communication, the communication unit 5059 is provided with a photoelectric conversion module that converts an optical signal into an electric signal. The communication unit 5059 provides the image processing unit 5061 with the image signal converted into an electric signal.

Furthermore, the communication unit 5059 transmits a control signal for controlling the driving of the camera head 5005 to the camera head 5005. The control signal may also be transmitted by optical communication.

The image processing unit 5061 performs various types of image processing on an image signal that is raw data transmitted from the camera head 5005. Examples of the image processing include various types of known signal processing such as development processing, high image quality processing (such as band emphasis processing, super-resolution processing, noise reduction (NR) processing, and/or camera shake correction processing), and/or enlargement processing (electronic zoom processing). Furthermore, the image processing unit 5061 performs demodulation processing on the image signal for performing AE, AF, and AWB.

The image processing unit 5061 is constituted by a processor such as a CPU or a GPU, and the image processing and demodulation processing described above can be performed by the processor operating in accordance with a predetermined program. Note that, in a case where the image processing unit 5061 is constituted by a plurality of GPUs, the image processing unit 5061 appropriately divides information related to the image signal, and image processing is performed in parallel by the plurality of GPUs.

The controller 5063 performs various controls related to capturing of an image of a surgical site by the endoscope 5001 and display of the captured image. For example, the controller 5063 generates a control signal for controlling the driving of the camera head 5005. At this time, in a case where imaging conditions have been input by a user, the controller 5063 generates a control signal on the basis of the input by the user. Alternatively, in a case where the endoscope 5001 has an AE function, an AF function, and an AWB function, the controller 5063 appropriately calculates an optimal exposure value, focal length, and white balance in accordance with a result of demodulation processing performed by the image processing unit 5061, and generates a control signal.

Furthermore, the controller 5063 causes the display device 5041 to display an image of a surgical site on the basis of an image signal on which image processing unit 5061 has performed image processing. At this time, the controller 5063 uses various image recognition technologies to recognize various objects in the image of the surgical site. For example, the controller 5063 can be recognize a surgical tool such as forceps, a specific living body site, bleeding, mist at the time of using the energy treatment tool 5021, and the like by detecting a shape, color, and the like of an edge of an object in the image of the surgical site. When displaying the image of the surgical site on the display device 5041, the controller 5063 superimposes various types of surgery support information upon the image of the surgical site using results of the recognition. By superimposing the surgery support information and presenting it to the operator 5067, surgery can be performed more safely and reliably.

The transmission cable 5065 connecting the camera head 5005 and the CCU 5039 is an electric signal cable that supports electric signal communication, an optical fiber cable that supports optical communication, or a composite cable thereof.

Here, in the illustrated example, wired communication is performed using the transmission cable 5065, but wireless communication may be performed between the camera head 5005 and the CCU 5039. In a case where wireless communication is performed between the two, the transmission cable 5065 does not need to be laid in the operating room. This may resolve a situation in which movement of medical staff in the operating room is hindered by the transmission cable 5065.

The example of the endoscopic surgery system 5000 to which the technology according to the present disclosure can be applied has been described above. Note that, although the endoscopic surgery system 5000 has been described as an example here, systems to which the technology according to the present disclosure can be applied are not limited to such an example. For example, the technology according to the present disclosure may be applied to an inspection flexible endoscope system or a microscopic surgery system.

The technology according to the present disclosure can be applied to, for example, a camera head among the configurations described above.

### REFERENCE SIGNS LIST

1, 1A, 1B, 1C, and 1D Distance measuring system
10, 10A, 10B, and 90 Optical member
11 Lens
12, 92 Bandpass filter
12A First filter
12B Second filter
12C Bandpass filter layer
12D Antireflection film
13, 13A Base material transparent to infrared light
14A Frame
14B Adhesive member
15, 15A Film sheet
16 Suction die
16A Concave portion
16B Opening
20, 20A, and 20B Light receiving unit
30, 30A, and 30B Analog-to-digital conversion unit
40, 40A, and 40B Arithmetic processing unit
50 Controller
60 Light source driving unit
70 Light source unit
71 Light diffusion member
72 Scanning unit
73 Pattern projection unit
80 Composition processing unit
120 Wafer-like bandpass filter group
140 Wafer-like frame
200 Wafer-like imaging element group

## Claims

1. A distance measuring system (1, 1A, 1B, 1C, 1D) comprising:
a light source unit (70) that is configured to emit infrared light toward a target object;
a light receiving unit (20) that is configured to receive the infrared light from the target object;
an arithmetic processing unit (40) that is configured to obtain information regarding a distance to the target object on a basis of data from the light receiving unit; and
an optical member (10, 10A, 10B) arranged on a light receiving surface side of the light receiving unit (20), wherein
the optical member (10, 10A, 10B) includes a bandpass filter (12),
the bandpass filter (12) comprises:
a first filter (12A) that is transparent to light in a predetermined wavelength range of infrared light; and
a second filter (12B) that is non-transparent to visible light and transparent to infrared light,
the first filter (12A) and the second filter (12B) are stacked and formed on one side of a base material (13, 13A), and
the bandpass filter (12) has a concave-shaped light incident surface.

2. The distance measuring system (1, 1A, 1B, 1C, 1D) according to claim 1, wherein
the optical member (10, 10A, 10B) comprises a lens (11) arranged on a light incident surface side of the bandpass filter (12), and
an incident angle of light at a maximum image height with respect to the light incident surface of the bandpass filter (12) is 10 degrees or less.

3. The distance measuring system (1, 1A, 1B, 1C, 1D) according to claim 1 or 2, wherein
a transmission band of the bandpass filter (12) has a half-width of 50 nm or less.

4. The distance measuring system (1, 1A, 1B, 1C, 1D) according to any one of the preceding claims, wherein
the first filter (12A) is arranged on a light incident surface side, and
the second filter (12B) is arranged on a light receiving unit side.

5. The distance measuring system (1, 1A, 1B, 1C, 1D) according to any one of the preceding claims, wherein
the arithmetic processing unit (40) is configured to obtain distance information on a basis of a time of flight of light reflected from the target object.

6. The distance measuring system (1, 1A, 1B, 1C, 1D) according to any one of preceding claims, wherein
infrared light is emitted in a predetermined pattern to the target object, and
the arithmetic processing unit (40) is configured to obtain distance information on a basis of a pattern of light reflected from the target object.

## Patentansprüche

1. Entfernungsmesssystem (1, 1A, 1B, 1C, 1D), umfassend:
eine Lichtquelleneinheit (70), die konfiguriert ist, um Infrarotlicht in Richtung eines Zielobjekts auszustrahlen;
eine Lichtempfangseinheit (20), die konfiguriert ist, um das Infrarotlicht vom Zielobjekt zu empfangen;
eine Recheneinheit (40), die konfiguriert ist, um Informationen hinsichtlich einer Distanz zum Zielobjekt auf Grundlage von Daten von der Lichtempfangseinheit zu erlangen; und
ein optisches Element (10, 10A, 10B), das auf einer Lichtempfangsoberflächenseite der Lichtempfangseinheit (20) angeordnet ist, wobei
das optische Element (10, 10A, 10B) einen Bandpassfilter (12) einschließt,
der Bandpassfilter (12) umfasst:
einen ersten Filter (12A), der für Licht in einem vorgegebenen Wellenlängenbereich von Infrarotlicht durchlässig ist; und
einen zweiten Filter (12B), der für sichtbares Licht undurchlässig und für Infrarotlicht durchlässig ist,
der erste Filter (12A) und der zweite Filter (12B) sind gestapelt und auf einer Seite eines Basismaterials (13, 13A) ausgebildet, und
der Bandpassfilter (12) weist eine konkav geformte Lichteinfallsfläche auf.

2. Entfernungsmesssystem (1, 1A, 1B, 1C, 1D) nach Anspruch 1, wobei
das optische Element (10, 10A, 10B) eine Linse (11) umfasst, die auf einer Lichteinfallsflächenseite des Bandpassfilters (12) angeordnet ist, und
ein Einfallswinkel des Lichts bei einer maximalen Bildhöhe in Bezug auf die Lichteinfallsfläche des Bandpassfilters (12) 10 Grad oder weniger beträgt.

3. Entfernungsmesssystem (1, 1A, 1B, 1C, 1D) gemäß Anspruch 1 oder 2, wobei
ein Transmissionsband des Bandpassfilters (12) eine Halbwertsbreite von 50 nm oder weniger aufweist.

4. Entfernungsmesssystem (1, 1A, 1B, 1C, 1D) nach einem der vorstehenden Ansprüche, wobei
der erste Filter (12A) auf einer Lichteinfallsflächenseite angeordnet ist, und
der zweite Filter (12B) auf der Seite einer Lichtempfangseinheit angeordnet ist.

5. Entfernungsmesssystem (1, 1A, 1B, 1C, 1D) nach einem der vorstehenden Ansprüche, wobei
die Recheneinheit (40) konfiguriert ist, um Entfernungsinformationen auf Grundlage der Flugzeit des vom Zielobjekt reflektierten Lichts zu erlangen.

6. Entfernungsmesssystem (1, 1A, 1B, 1C, 1D) gemäß einem der vorhergehenden Ansprüche, wobei
Infrarotlicht in einem vorgegebenen Muster auf das Zielobjekt ausgestrahlt wird und
die Recheneinheit (40) konfiguriert ist, um Entfernungsinformationen auf Grundlage eines Musters des vom Zielobjekt reflektierten Lichts zu erlangen.

## Revendications

1. Système de mesure de distance (1, 1A, 1B, 1C, 1D) comprenant :
une unité de source de lumière (70) qui est configurée pour émettre une lumière infrarouge en direction d'un objet cible ;
une unité de réception de lumière (20) qui est configurée pour recevoir la lumière infrarouge provenant de l'objet cible ;
une unité de traitement arithmétique (40) qui est configurée pour obtenir des informations concernant une distance par rapport à l'objet cible sur la base de données provenant de l'unité de réception de lumière ; et
un élément optique (10, 10A, 10B) agencé sur un côté surface de réception de lumière de l'unité de réception de lumière (20), dans lequel
l'élément optique (10, 10A, 10B) comporte un filtre passe-bande (12),
le filtre passe-bande (12) comprend :
un premier filtre (12A) qui est transparent à la lumière dans une gamme prédéterminée de longueurs d'onde de la lumière infrarouge ; et
un second filtre (12B) qui est non transparent à la lumière visible et transparent à la lumière infrarouge,
le premier filtre (12A) et le second filtre (12B) sont empilés et formés sur une face d'un matériau de base (13, 13A), et
le filtre passe-bande (12) a une surface d'incidence de lumière de forme concave.

2. Système de mesure de distance (1, 1A, 1B, 1C, 1D) selon la revendication 1, dans lequel
l'élément optique (10, 10A, 10B) comprend une lentille (11) agencée sur un côté surface d'incidence de lumière du filtre passe-bande (12), et
un angle d'incidence de la lumière à une hauteur d'image maximale par rapport à la surface d'incidence de lumière du filtre passe-bande (12) est de 10 degrés ou moins.

3. Système de mesure de distance (1, 1A, 1B, 1C, 1D) selon la revendication 1 ou 2, dans lequel
une bande de transmission du filtre passe-bande (12) a une demi-largeur de 50 nm ou moins.

4. Système de mesure de distance (1, 1A, 1B, 1C, 1D) selon l'une quelconque des revendications précédentes, dans lequel
le premier filtre (12A) est agencé sur un côté surface d'incidence de lumière, et
le second filtre (12B) est agencé sur un côté unité de réception de lumière.

5. Système de mesure de distance (1, 1A, 1B, 1C, 1D) selon l'une quelconque des revendications précédentes, dans lequel
l'unité de traitement arithmétique (40) est configurée pour obtenir des informations de distance sur la base d'un temps de vol de la lumière réfléchie par l'objet cible.

6. Système de mesure de distance (1, 1A, 1B, 1C, 1D) selon l'une quelconque des revendications précédentes, dans lequel
la lumière infrarouge est émise selon un motif prédéterminé vers l'objet cible, et
l'unité de traitement arithmétique (40) est configurée pour obtenir des informations de distance sur la base d'un motif de lumière réfléchi par l'objet cible.
